# EUROPEAN PATENT APPLICATION

(11) **EP 4 317 446 A1**
(43) Date of publication of application: **07.02.2024**
(21) Application number: 22781492.8
(22) Date of filing: 24.03.2022
(51) Int. Cl.: C12N 15/86, C12N 15/85, A61P 31/14

(54) **RECOMBINANT CHIMERIC ADENOVIRAL VECTOR SUBSTITUTED BY KNOB GENE OF CHIMPANZEE ADENOVIRUS SEROTYPE 6, AND APPLICATION THEREOF**

(30) Priority: 29.03.2021 KR 20210040269; 21.03.2022 KR 20220034885
(71) Applicant: GENEMATRIX INC., Seongnam-si, Gyeonggi-do 13488 (KR)
(72) Inventor: KIM, Soo-Ok, Seongnam-si, Gyeonggi-do 13105 (KR); HONG, Sun Pyo, Seoul 06715 (KR); KIM, Suk Joon, Seongnam-si, Gyeonggi-do 13532 (KR); JANG, Sun Ok, Seoul 06959 (KR); KIM, Hyun Ju, Yongin-si, Gyeonggi-do 16875 (KR); LEE, Sang Hyun, Seongnam-si, Gyeonggi-do 13438 (KR)
(74) Representative: FRKelly
(86) International application number: PCT/KR2022/004159
(87) International publication number: WO 2022/211373

(57) **Abstract**

The present invention is a chimeric adenovirus vector in which the knob domain of the end of the fiber protein of human adenovirus type 5 is replaced with the knob gene of chimpanzee adenovirus serotype 6 and/or in addition the hexon protein of human adenovirus type 5 is replaced with hypervariable regions 1-7 of human adenovirus serotype 28. The present invention not only provides the optimal adenovirus vector in the development of treatments or vaccines for various diseases, but also when the chimeric adenovirus vector produced in the present invention is infected with a host cell for production, it can contribute to improved productivity by exhibiting superior cell infection ability compared to the recombinant HAdV-5 vector-based vaccine.

## Description

### [Technical Field]

The present invention relates to a chimeric adenovirus vector having optimal conditions for avoiding pre-existing immunity against human adenovirus serotype 5 (HAdV-5) in a host. Specifically, it relates to a chimeric adenovirus vector expressing the knob protein and/or hexon protein of an adenovirus serotype with low seroprevalence based on the HAdV-5 gene sequence, and prophylactic and therapeutic vaccine applications against SARS-CoV-2 and human papillomavirus based on the chimeric adenovirus vectors.

### [Background Art]

Adenovirus is a medium-sized nonenveloped virus with a diameter of about 90-100 mm containing linear double stranded DNA in a nucleocapsid. It is found in a variety of vertebrates (amphibians, birds, and mammals), most of which cause respiratory infections, conjunctivitis and gastroenteritis. In contrast to retroviruses, since it is not inserted into the genome of host cells, transformation of host cells or tumorigenesis by adenovirus DNA does not occur, transduction into non-dividing cells is possible, and virus productivity is high. Because of their characteristics, adenoviral vectors have been widely studied for gene therapy and vaccine applications.

First-generation recombinant adenovirus vectors delete E1 and E3 genes, which are early transcription genes involved in viral replication, and from a clinical point of view, they are replication-incompetent in the host. side effects were reduced by making it, and there were fewer restrictions on inserting a foreign gene (up to about 8 Kb). So far, human adenovirus serotypes 2 and 5 (AdH2 and HAdV-5) belonging to subgroup C have been studied most as vectors, and recombinant HAdV-5 vector-based vaccines have been developed in preclinical trials. Excellent vaccine efficacy was demonstrated by showing highly immunogenic results through various animal models.

However, a clinical trial of an HIV vaccine developed based on a recombinant HAdV-5 vector showed insufficient efficacy different from non-clinical results. HAdV-5 is one of the most infected adenovirus serotypes, and it has been reported that about 80% or more of the population has anti-HAdV-5 neutralizing antibodies due to pre-existing immunity. As shown in the results of HIV vaccine clinical trials, anti-HAdV-5 neutralizing antibodies formed by preexisting immunity bind to recombinant HAdV-5 vector-based vaccines loaded with foreign genes, reducing vaccine efficacy.

The icosahedral envelope protein of adenovirus is the main capsid protein and is composed of hexon, fiber and penton proteins. Hexon is a protein that occupies the largest portion of adenovirus envelope proteins and includes a hypervariable region (HVR) with low genetic homology between adenovirus serotypes. Penton is a protein responsible for the structural stability and flexibility of envelope proteins and is involved in cell entry through the Arg-Gly-Asp (RGD) motif. In addition, the fiber binds to the host cell's receptor, CAR (Coxsackie and adenovirus receptor), through a knob domain at the end, and cell entry occurs. Three coat proteins (hexon, fiber and penton) are known to act as major recognition sites for anti-Ad neutralizing antibodies. Recently, to avoid existing immunity to HAdV-5, a chimeric adenovirus vector study was reported in which the projection domain of the fiber protein was replaced with a human adenovirus serotype with low seroprevalence. However, in the case of chimeric adenoviral vectors using human adenovirus serotypes, there is a possibility of cross-reactivity with neutralizing antibodies against HAdV-5, so the ability to evade existing immunity against HAdV-5 may be reduced.

To avoid cross-reactive immunity with neutralizing antibodies to HAdV-5, adenovirus serotypes isolated from non-human animals that are genetically distant have been developed as viral vectors. Among these non-human adenovirus vectors, a viral vector using a chimpanzee adenovirus (ChAdV) serotype is most widely used. ChAdV serotypes were investigated to have lower seroprevalence rates than HAdV-5. For example, the serum retention rates of neutralizing antibodies against ChAdV-6 and ChAdV-68 were detected in only 1.5-4% of serum samples from the US and Thailand. In addition, in the phase 1 clinical trial of the ChAdV-63-based malaria vaccine, safety and antigen-specific T cell response were high in all clinical trial volunteers. Despite the low seroprevalence of ChAdV-vector-based vaccines, high cross-reactivity between serotypes is a major concern for vector-specific cellular immunity.

Coronaviruses are positive-sense single-stranded RNA viruses that have an envelope on the surface of particles with a diameter of 100-200 mm. Seven types of coronaviruses are known to cause respiratory and digestive infections in humans, and two of the seven types are alpha- genus of coronaviruses, and the remaining five species were identified as genus beta-coronaviruses. Severe acute respiratory syndrome coronavirus 2 (hereinafter referred to as 'SARS-CoV-2'), which causes COVID-19 (novel coronavirus, 2019-nCov), is a beta-coronavirus. After it was first reported in Wuhan City, China in December 2019 (Wuhan-Hu-1), confirmed cases are appearing one after another around the world, and the pandemic is intensifying through various mutations (alpha variants, beta variants, delta variants, etc.) since the outbreak.

The viral envelope of SARS-CoV-2 is composed of spike glycoprotein (S protein) and hemagglutinin-esterase dimer (HE) etc.. The S 1 region of the S protein mainly binds to the receptor (Angiotensin-converting enzyme 2, ACE2) through the RBD (Receptor binding domain), and the S2 region is responsible for direct cell-viral fusion (initiation of infection) with dramatic changes in tertiary structure. Therefore, vaccine candidates targeting the S protein as a major antigen have been developed and commercialized (AZD-1222 (AstraZeneca), Bnt162b2 (Pfizer), mRNA-1273 (Moderna) etc.)

Unlike DNA viruses, which have DNA repair pathways (base excision repair, nucleotide excision repair, mismatch repair) that correct errors during DNA replication, RNA viruses use RNA polymerase, and so they do not have RNA repair-related proteins to correct errors generated during genome replication, so they are characterized by frequent mutations compared to DNA viruses. In the case of the SARS-CoV-2 variant virus, mutations frequently occurred in the N-terminal domain (NTD) and RBD domain of the S protein, as a result, licensed vaccines developed targeting the S protein of SARS-coronavirus-2 have reduced antibody binding ability to the S protein of the variant virus in which structural changes have occurred, resulting in reduced protective efficacy against the variant virus.

In December 2020, a fatal mutation of SARS-CoV-2 occurred in South Africa and was classified as a beta mutation and designated as a variant of concern (VOC). The mutant was found to be the first mutant to have all three key mutations (E484K, K417N, N501Y) of VOCs in RBD as well as deletion in NTD, and in the case of AstraZeneca's AZD-1222 vaccine, it was confirmed that the efficacy of the vaccine in the beta variant strain (B.1.3 51) group was reduced by 10% compared to the existing SARS-CoV-2, and a rapid decline in efficacy against licensed vaccines.

In addition, as cross-neutralization was confirmed for SARS-coronavirus-2 and other variant viruses in the serum of convalescent patients who were infected with the beta mutation, the direction is changing towards the development of a vaccine targeting the S protein against the variant virus, not the S protein against SARS-CoV-2, and the need for a universal vaccine that can fight against the continuous emergence of variant viruses is emerging.

HPV has about 170 HPV genotypes, which are largely classified into two groups: high risk and low risk. HPV-6/11/40/42/43/44/54/61 and 72 types are in the low-risk group for causing genital warts, and HPV-16/18/31/35/39/45/51/ Including 52/56/58/66 and 68, the high-risk type is responsible for about 99.7% of cervical cancer cases.

Particularly, HPV-16 and 18 types are the most common types associated with cervical cancer worldwide, accounting for more than 70% of cases. HPV is also responsible for many penile, vulvar, and anal-related carcinomas and accounts for more than 40% of oropharyngeal cancers.

Persistent infection with these high-risk HPVs results in the development of squamous intraepithelial lesions (SILs). This is called cervical intraepithelial neoplasia (CIN) in the cervix and vulva intraepithelial neoplasia (VIN) in the vulva, and these SILs can progress to malignant cancer.

HPV is a non-enveloped virus consisting of double-stranded DNA with a genome size of approximately 8 kb. The genome encodes six early regulatory proteins (E1, E2, E4, E5, E6 and E7) and two late structural proteins (L1 and L2). Early genes encode proteins involved in viral DNA replication, transcription and oncogenic transformation, and late genes encode viral capsid proteins. In the general high-risk HPV cancer formation process, the viral genome is integrated into the host's chromosomal DNA, and the E2 genetic sequence is disrupted during gene linearization. Since E2 protein is a transcriptional repressor of E6 and E7, disruption of E2 initiates the expression of E6 and E7 proteins. The E6 protein promotes the degradation of p53, a protein that regulates apoptosis of the host, and activates telomerase to extend cell lifespan. E7 protein also targets and degrades the tumor suppressor protein retinoblastoma protein (pRb). As such, E6 and E7 interfere with cell cycle regulation and extend the lifespan of host cells, causing genetic instability, malignant transformation of cells, and cancer.

In HPV therapeutic vaccines, infection elimination in cellular immunity is more important than humoral immune response. HPV E6 and E7 oncoproteins are essential for the initiation and maintenance of malignant tumors and are expressed in pre-malignant and invasive lesions but absent from healthy cells.

Therefore, these oncoproteins (E6, E7) cannot avoid immune responses due to mutations, and are the most ideal targets for the development of therapeutic vaccines against HPV infections and lesions.

An ideal therapeutic vaccine can target E6 and E7 proteins to induce strong tumor-specific T cell type 1 and cytotoxic T lymphocyte (CTL) responses to kill infected and malignant cells.

### [Prior Patent Literature]

Korean Patent Publication No. 1020040054796

### [Disclosure]

### [Technical Problem]

The present invention has been made in response to the above needs, and an object of the present invention is to provide an adenoviral vector that is superior to conventional recombinant HAdV-5 vectors in terms of constructing a chimeric adenoviral vector that overcomes pre-existing immunity to HAdV-5 in the host.

Another object of the present invention is to provide a therapeutic or prophylactic vaccine vector capable of delivering foreign genes with high efficiency.

Another object of the present invention is to provide a chimeric adenovirus vector comprising the S gene sequence of the beta variant to prevent infection with SARS-coronavirus-2.

Another object of the present invention is to provide a vaccine for preventing infection of SARS-coronavirus-2.

Another object of the present invention is to provide a chimeric adenovirus vector comprising gene sequences of 16 types E6 and E7 and 18 types E6 and E7 of human papillomavirus.

Another object of the present invention is to provide a vaccine for the treatment and prevention of HPV.

### [Technical Solution]

In order to achieve the above object, the present invention provides a chimeric adenoviral vector based on human adenovirus serotype 5 having excellent ability to evade existing immunity that the knob domain at the end of the fiber protein of human adenovirus type 5 is substituted with the knob gene of chimpanzee adenovirus serotype 6, or the hexon protein of human adenovirus type 5 is substituted with hypervariable regions 1-7 of human adenovirus serotype 28 in the substituted type 5 adenovirus.

The present invention relates to a vector capable of avoiding pre-existing immunity to human adenovirus type 5 to overcome the decrease in efficacy when exogenous gene transfer due to existing immunity in humans against human adenovirus type 5.

In the present invention, the replacement site of the HAdV-5 gene is the knob domain of the fiber protein. Fiber proteins are composed of three domains (tail, shaft, and knob), among which the knob domain is known as a major recognition site for neutralizing antibodies.

In one embodiment of the present invention, the protein expressed by the chimpanzee adenovirus serotype 6 knob gene preferably includes the amino acid sequence of SEQ ID NO: 1 but is not limited thereto.

In another embodiment of the present invention, the knob gene sequence of the chimpanzee adenovirus serotype 6 preferably comprises the nucleic acid sequence of SEQ ID NO: 2 but is not limited thereto.

The knob sequence of the present invention uses the gene sequence of a rare adenovirus serotype having a low serum retention rate in humans. For example, there are ChAdV-1, ChAdV-2, ChAdV-3, ChAdV-5, ChAdV-7, ChAdV-68, etc. In addition to chimpanzee adenovirus, adenovirus serotypes found in cattle, pigs, birds, etc. can be used.

The present invention provides a chimeric adenovirus vector comprising the tail and shaft domains of human adenovirus type 5 and the knob combined fiber protein of chimpanzee adenovirus serotype 6.

In another embodiment of the present invention, the chimeric protein in which the hexon protein of human adenovirus type 5 is substituted with hypervariable regions 1-7 of human adenovirus serotype 28 has the amino acid sequence of SEQ ID NO: 3, The hypervariable region gene sequence in the hexon protein of the human adenovirus serotype 28 preferably comprises the nucleic acid sequence of SEQ ID NO: 4 but is not limited thereto.

The hexon hypervariable region sequence of the present invention uses the gene sequence of a rare adenovirus serotype having a low serum retention rate in humans. For example, there are HAdV-14, HAdV-19, HAdV-32, HAdV-34, HAdV-36, HAdV-43, etc., and adenovirus serotypes found in cattle, pigs, birds, etc. besides humans can be used.

In the present invention, a chimeric adenoviral vector comprising a hexon protein obtained by replacing a hypervariable region in a hexon protein of human adenovirus type 5 with a gene of human adenovirus type 28 in an adenovirus vector comprising a fiber protein combined with the knob gene of chimpanzee adenovirus serotype 6 is provided.

The vector of the present invention is characterized in that it comprises Inverted terminal repeat (ITR) sequence, BR322 origin of replication, kanamycin resistance gene, ITR sequence, encapsidation signal, CMV enhancer, CMV promoter, and SV40 ploy (A) signal as vector regulators of the present invention.

The vector preferably comprises a BR322 origin of replication and an antibiotic resistance gene, and preferably uses a kanamycin resistance gene (Kan) but is not limited thereto. Antibiotic resistance genes for ampicillin, chloramphenicol, tetracycline, or neomycin may be used as antibiotics used for selection.

The vector preferably comprises an inverted terminal repeat (ITR) sequence and an encapsidation signal of adenovirus. ITR sequences and encapsidation sequences are essential elements for adenoviral DNA replication and packaging, respectively.

The vector preferably comprises, but is not limited to, a cytomegalovirus immediate early enhancer (CMV enhancer) as an enhancer. For example, an enhancer of Simian virus 40 (SV40) may be used as an enhancer.

The promoter is preferably a virus-derived or mammalian-derived promoter, and specifically refers to a cytomegalovirus (CMV) promoter. The CMV promoter is recognized as a powerful promoter that efficiently induces exogenous gene expression in all mammalian and insect cells.

The SV40 poly(A) signal sequence of the vector refers to the poly adenyl signal of Simian virus 40 (SV40) and is preferably comprised in the vector. The use of the SV40 poly(A) signal sequence increases the stability of messenger RNA (mRNA), making protein expression easier.

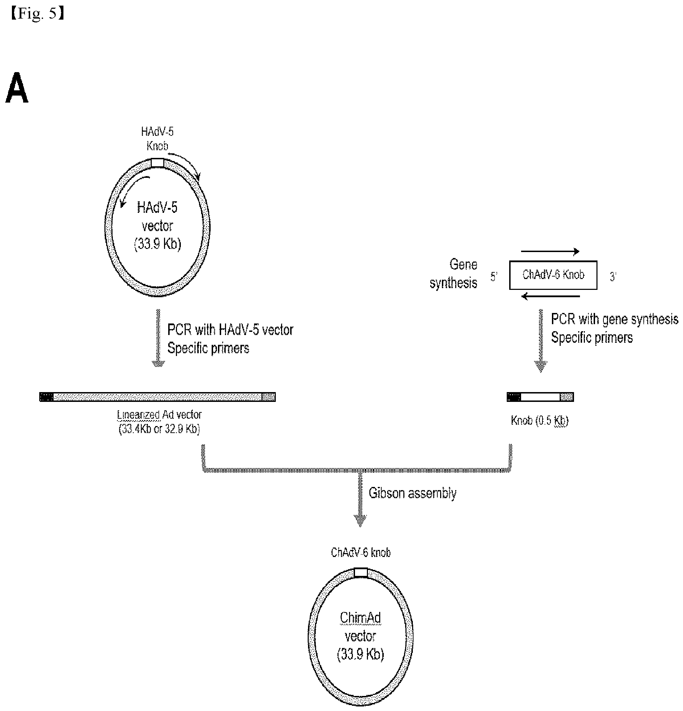

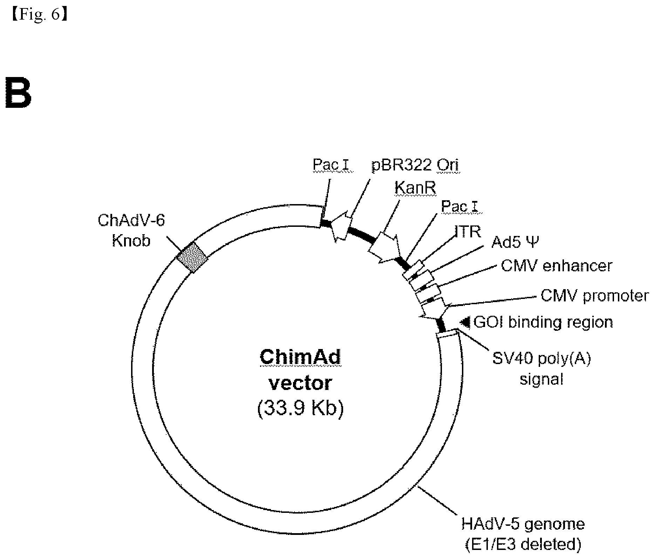

The vector of the present invention is preferably the chimeric adenoviral vector shown in FIG. 5 or 6 but is not limited thereto.

In one embodiment of the present invention, the vector preferably comprises a) a gene encoding the coronavirus spike protein, or b) a gene encoding the E6 and E7 proteins of human papillomavirus type 16 or 18 as an insert but is not limited to thereto.

In another embodiment of the present invention, the vector is preferably capable of infecting mammalian cells but is not limited thereto.

The present invention also provides a host cell transformed with the chimeric adenoviral vector of the present invention.

In one embodiment of the present invention, the transformed cell of the chimeric adenoviral vector that substituted with the chimpanzee adenovirus knob gene and the chimeric adenoviral vector substituted with the hexon hypervariable region of a rare human adenovirus is preferably mammalian cells including HEK293, PERC6 or 911 cells, more preferably HEK293 cells but are not limited thereto.

For example, an adenovirus-producing cell suitable for the present invention should be a cell capable of producing a virus by trans-complementation of the E1 and E3 gene deletion sites of a replication defective adenovirus, and many cells transformed with the viral early transcription genes are being developed. Preferred cells into which the E1 and E3 genes are introduced comprise A549 (carcinomic human alveolar basal epithelial cell), HeLa (Human cervical cancer cell), CHO (Chinese hamster ovary cell), MRC5 (human lung fibroblast), and PC12 (rat pheochromocytoma cell) etc but not limited thereto.

The present invention also provides an isolated polypeptide encoded by the chimeric adenoviral vector of the present invention.

In addition, the present invention provides a vaccine composition comprising the chimeric adenovirus vector of the present invention or a polypeptide encoded by the vector as an active ingredient.

In the present invention, the above elements are artificially synthesized and provide a vector through the Gibson assembly method.

In an embodiment of the present invention, the chimeric adenovirus vector-based vaccine is preferably beta variant S or human papillomavirus types 16 and 18 E6E7 proteins but is not limited thereto.

In addition, the present invention provides mouse immunity and virus neutralization efficacy by using the beta variant strain S based on the chimeric adenovirus vector.

In addition, the present invention provides the efficacy for the immune response of specific T cells according to the beta variant strain S based on the chimeric adenovirus vector.

The present invention provides protective efficacy against SARS-coronavirus-2 and delta variant virus after immunization with beta variant S virus based on chimeric adenovirus vector.

The present invention provides the efficacy of mouse tumor cell therapy using HPV16 and 18 E6E7 viruses based on the chimeric adenovirus vector.

The present invention provides efficacy against the immune response of specific T cells against HPV16 and 18 E6E7 viruses based on a chimeric adenovirus vector.

The present invention provides the efficacy of mouse tumor cell prevention using HPV16 and 18 E6E7 viruses based on chimeric adenovirus vectors.

In addition, the present invention provides a vaccine composition for preventing or treating an infection of SARS-CoV-2 by the chimeric adenovirus vector of the present invention or being encoded by the vector.

In addition, the present invention provides a composition characterized in that it is a vaccine for treating cervical cancer of human papillomavirus disease by the chimeric adenovirus vector of the present invention or being encoded by the vector.

The present invention will be described below.

The present inventors have made diligent efforts to develop a chimeric adenovirus vector that improves the existing immunity problems against HAdV-5 by combining the capsid protein gene sequences of adenovirus serotypes with low serum retention, and as a result, the present invention was completed by confirming that the ChimAd vector in which the knob gene sequence was replaced with the corresponding gene of ChAdV-6 based on the recombinant HAdV-5 vector, or ChimAd-H28 vector in which the hypervariable region in the hexon protein of HAdV-5 was replaced with the corresponding gene of serotype 28 can be usefully used as an optimal adenoviral vector..

Since more than 80% of the population naturally possesses neutralizing antibodies against adenovirus, vaccines based on adenovirus vectors loaded with foreign genes have a problem in that immune efficacy is reduced due to existing immunity against HAdV-5. The developed chimeric adenoviral vector presents the potential as an excellent adenoviral vector that improves these problems of the conventional recombinant HAdV-5 vector through cytopathic effect (CPE) and in vitro neutralizing antibody tests..

Specifically, the present invention provides an AdKnob vector in which the gene sequence of the HAdV-5 knob domain is replaced with the corresponding gene of chimpanzee adenovirus serotype 6, a rare serotype, based on a recombinant vector.is. based on a recombinant HAdV-5 vector composed of a BR322 origin of replication, a kanamycin resistance gene, an inverted terminal repeat (ITR) sequence, an encapsidation signal, a CMV enhancer, a CMV promoter, and a SV40 poly(A) signal sequence.

In addition, the present invention provides a ChimAd-H28 vector in which a region in the hexon protein of HAdV-5 is replaced with a corresponding gene of HAdV-28, an adenovirus serotype with low human seroprevalence, based on the ChimAd vector.

The present invention provides the existing immune avoidance effect by testing the cell infectivity and neutralizing antibody for the ChimAd and ChimAd-H28 vectors.

To analyze the cell infectivity of the chimeric adenovirus provided in the present invention, compared to the existing HAdV-5, the virus titer was measured under the same MOI virus dose (multiplicity of infection) and time conditions. As a result, it was confirmed that the number of cells infected with the chimeric adenovirus and the virus titer increased more than the HAdV-5 virus. Due to the knob gene of ChAdV-6 and the HVR gene of HAdV-28, the ability to infect host cells increased, and the virus productivity increased, compared to HAdV-5.

In addition, in the present invention, the neutralizing antibody immunity evasion ability of the ChimAd vector against HAdV-5 was evaluated through an in vitro neutralizing antibody test. In the in vitro neutralizing antibody test, anti-HAdV-5 antibody and virus were reacted, were infected to HEK293 cells , and finally cells infected with HAdV-5, ChimAd and ChimAd-H28 viruses were identified. As a result, when compared with the HAdV-5 virus, about 4 times more adenovirus-infected cells were confirmed in 10¹-10² by the ChimAd virus, indicating a higher ability to evade existing immunity than the conventional HAdV-5. In addition, about 2.8 times more infected cells were identified by the ChimAd-H28 virus.

The present invention provides a composition comprising an immunological adjuvant, the chimeric vector or a polypeptide encoded by the vector, and optionally a pharmaceutically acceptable excipient.

A composition according to the invention comprising an adjuvant can be used, for example, as a vaccine for human subjects. Immunological adjuvants, also referred to herein as "adjuvants" for short, accelerate, prolong and/or enhance the quality and/or strength of the immune response to the antigen/immunogenicity, compared to administration of the antigen alone. This reduces the amount of antigen/immunogen required for any given vaccine and/or the frequency of injections required to generate an adequate immune response to the desired antigen/immunogen.

Examples of adjuvants that may be used in connection with the composition according to the present invention are gel-like precipitates of aluminum hydroxide (allum); AlPO₄; alhydrogel; bacterial products from the outer membrane of Gram-negative bacteria, in particular monophosphoryl lipid A (MPLA), lipopolysaccharide (LPS), muramyldipeptide and derivatives thereof; Freund's incomplete adjuvant; liposomes, particularly neutral liposomes, liposomes containing a composition and optionally a cytokine; non-ionic block copolymers; ISCOMATRIX adjuvant (Drane et al, 2007); unmethylated DNA containing CpG dinucleotides (CpG motifs), in particular CpG ODNs having a phosphorothioate (PTO) backbone (CpG PTO ODN) or phosphodiester (PO) backbones (CpG PO ODN); synthetic lipopeptide derivatives, particularly Pam3Cys; lipoarabinomannan; peptidoglycan; zymosan; heat shock proteins (HSP), especially HSP 70; dsRNA and its synthetic derivatives, particularly Poly Lpoly C; polycationic peptides, especially poly-L-arginine; taxol; fibronectin; flagellin; imidazoquinoline; cytokines with adjuvant activity, in particular GM-CSF, interleukin-(IL-)2, IL-6, IL-7, IL-18, type I and type II interferons, in particular interferon-gamma, TNF-alpha; 25-dihydrophytamine D3 (calcitriol); and synthetic oligopeptides, particularly MHCII-provided peptides. Non-ionic block polymers containing polyoxyethylene (POE) and polyoxypropylene (POP), for example POE-POP-POE block copolymers, can be used as adjuvants (Newman et al, 1998). Adjuvants of this type are particularly useful for compositions that include nucleic acids as active ingredients.

If desired, various pharmaceutically acceptable excipients may be used. Preferred pharmaceutically acceptable excipients are mentioned below when discussing the use according to the present invention.

Activation of specific receptors can stimulate an immune response. Such receptors are known to the person skilled in the art and include, for example, cytokine receptors, especially type I cytokine receptors, type II cytokine receptors, TNF receptors; and vitamin D receptors that act as transcription factors; and toll-like receptor 1 (TLR1), TLR-2, TLR 3, TLR4, TLR5, TLR-6, TLR7, and TLR9. Agonists for these receptors have adjuvant activity, ie are immunostimulatory.

According to an embodiment of the present invention, ChimAd-CV and ChimAd-CVN comprising the S sequence of the beta variant strain based on the ChimAd vector and the N partial sequence of SARS-coronavirus-2 are provided.

In another embodiment of the present invention, the modified spike protein of the beta variant strain of SARS-coronavirus-2 is preferably comprised of the amino acid sequence shown in SEQ ID NO: 7, and the modified spike protein of the beta variant strain of SARS-coronavirus-2 The gene encoding the modified spike protein of the beta variant strain of SARS-coronavirus-2 is preferably composed of the nucleotide sequence shown in SEQ ID NO: 8 but all mutant sequences that achieve the object of the present invention through mutation of one or more substitutions, deletions, inversions, etc. to the sequence are also included in the scope of the present invention.

In one embodiment of the present invention, the amino acid residues from positions 44 to 180 of the nucleocapsid protein of the SARS-coronavirus-2 is shown in the amino acid sequence of SEQ ID NO: 9, and the amino acid residues from positions 247 to 364 of the nucleocapsid protein is preferably shown in the amino acid sequence of SEQ ID NO: 10, but all mutant sequences that achieve the object of the present invention through mutation such as one or more substitutions, deletions, inversions, etc. included in the scope of the invention.

The ChimAd-CV and ChimAd-CVN vaccine compositions produced in the present invention provide excellent neutralizing antibody titers against SARS-coronavirus-2, beta variants and delta variants.

The ChimAd-CV and ChimAd-CVN vaccine compositions produced according to an embodiment of the present invention provide excellent neutralizing antibody titers against pseudo-viruses based on beta variants.

The ChimAd-CV and ChimAd-CVN vaccine compositions produced in the present invention provide efficacy against the immune response of specific T cells.

In the present invention, the ChimAd-CV and ChimAd-CVN vaccine compositions produced by the above method provide protective efficacy against SARS-coronavirus-2 and delta variants.

Body weight change and survival rate after immunization and viral infection with ChimAd-CV and ChimAd-CVN produced according to an embodiment of the present invention were confirmed. Both experimental groups inoculated with ChimAd-CV and ChimAd-CVN against SARS-coronavirus-2 infection showed a 100% survival rate, confirming excellent defense. In addition, for delta variant infection, ChimAd-CV maintained 100% survival rate and 100% average body weight after 7 days of infection, but ChimAd-CVN showed a survival rate of 40% and average weight loss within about 20% after 7 days of infection.

According to an embodiment of the present invention, ChimAd-HPV comprising E6E7 and type 18 E6E7 sequences of human papillomavirus type 16 based on a ChimAd vector is provided.

In another embodiment of the present invention, the human papillomavirus type 16 E6E7 and type 18 E6E7 proteins are preferably comprised of the amino acid sequence shown in SEQ ID NO: 13, and The gene encoding the modified E6 and E7 of the human papillomavirus type 16 E6E7 and type 18 are preferably composed of the nucleotide sequence shown in SEQ ID NO: 14, but all mutant sequences that achieve the object of the present invention through mutation such as one or more substitutions, deletions, inversions, etc. are also included in the scope of the present invention.

In the present invention, to confirm the efficacy of human papillomavirus in treating cervical cancer, TC-1 tumor cells and the produced virus were injected. According to an embodiment of the present invention, the therapeutic efficacy was confirmed by measuring the tumor volume after injection of the recombinant antigen. As a result, the therapeutic efficacy of the ChimAd-HPV virus on tumor volume compared to the control group (ChimAd) was confirmed.

In addition, the ChimAd-HPV vaccine composition produced according to the embodiment of the present invention provides efficacy against the immune response of specific T cells.

To confirm the immune response efficacy of the specific T cells of virus produced in the present invention, ELISPOT (Enzyme-Linked ImmunoSpot, ELISPOT) experimental technique was used. In the present invention, tumor cells and antigen-injected mouse splenocytes were separated to confirm the immune response to each antigen. As a result, it is thought that the ChimAd-HPV virus induces a specific immune response against the HPV E7 protein antigen.

The present invention provides the efficacy of preventing mouse tumor cells with respect to the ChimAd-HPV vaccine composition produced by the above method. In the present invention, to confirm the preventive effect of cervical cancer by human papillomavirus, the virus and TC-1 tumor cells were injected. In the present invention, after injecting the recombinant antigen, tumor cells were injected to confirm the preventive effect on cancer. As a result, it was confirmed that the ChimAd-HPV virus did not generate tumor cells compared to the control group.

### [Advantageous Effects]

As can be seen through the present invention, the present invention can provide an optimal adenoviral vector in the development of a therapeutic agent or vaccine for various diseases. In addition, when the chimeric adenoviral vector constructed in the present invention is infected with host cells for production, it can contribute to productivity improvement by exhibiting superior cell infectivity compared to recombinant HAdV-5 vector-based vaccines. In addition, the chimeric adenovirus of the present invention can overcome existing immunity to HAdV-5 when inoculated into humans. In addition, it is considered that it will be an excellent chimeric adenovirus vector as the immune effectiveness that can more effectively transfer foreign genes is confirmed when developing a preventive or therapeutic vaccine for SARS-coronavirus-2 and human papillomavirus.

### [Description of Drawings]

Figure 1 shows the results of sequence analysis of the amino acid sequences of the fiber proteins of HAdV-5 and ChAdV-6 in the present invention through Clustal Omega and ESPript programs (A) and a schematic diagram of the fiber proteins of the chimeric adenovirus vector (B),
Figure 2 shows the results of sequence analysis of the amino acid sequences of the hexon proteins of HAdV-5 and HAdV-28 in the present invention through Clustal Omega and ESPript programs (A) and a schematic diagram of the hexon proteins of the chimeric adenovirus vector (B),
Figure 3 schematically shows the sequence of the vector regulator synthetic gene produced in the present invention,
Figure 4 is a schematic diagram showing the HAdV-5 vector construction process in the present invention,
Figures 5 and 6 are schematic diagrams of the ChimAd vector production process (Fig. 5) and the final ChimAd vector structure (Fig. 6) produced in the present invention.,
Figures 7 and 8 are schematic diagrams showing the production process of the ChimAd-H28 vector (Fig. 7) and the final structure of the ChimAd-H28 vector (Fig. 8) produced in the present invention,
Figure 9 shows the result of confirming the chimeric capsid gene in the two chimeric adenovirus vectors (ChimAd, ChimAd-H28) prepared in the present invention by amplifying the knob gene (0.5 Kb) of ChAdV-6 and the hexon HVR gene of HAdV-28 (1 Kb) by PCR, separating them on a 0.8% agarose gel, and passing ultraviolet rays through them,
Figure 10 is a photograph at 200x magnification using an optical microscope to show the formation of cytopathic effect (CPE) after induction of transformation in HEK293 cells with the HAdV-5 vector and two types of chimeric adenovirus vectors prepared in the present invention,
Figure 11 shows the expression of hexon (about 108 kDa), fiber (about 61 kDa), and penton (about 68 kDa) proteins through Western blot after HAdV-5 and two kinds of chimeric adenovirus were produced in HEK293 cells,

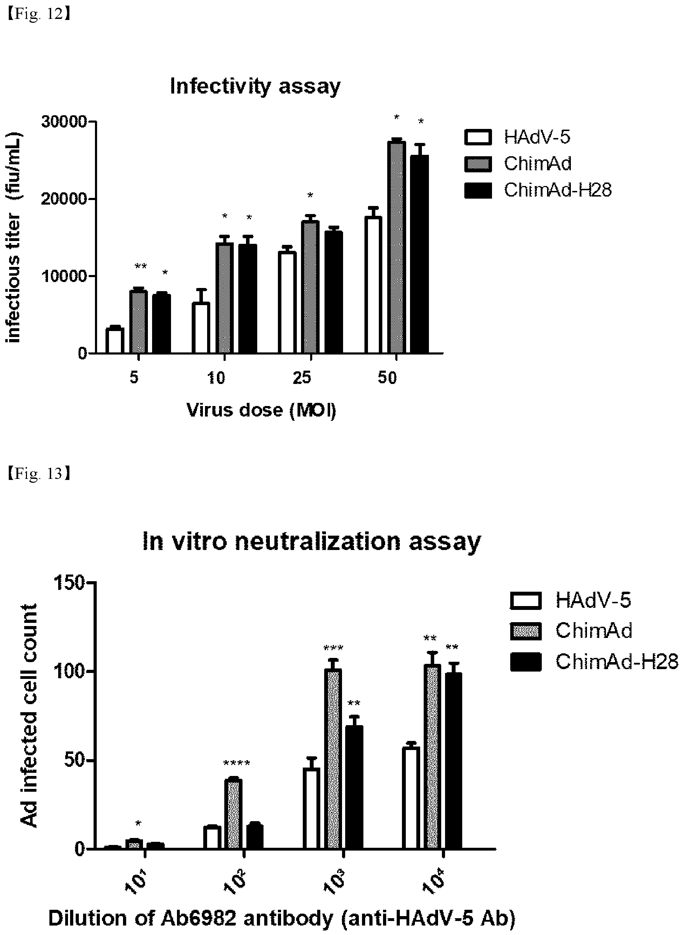

Figure 12 is a graph showing the number of positive cells infected with adenovirus taken at 200-fold magnification of an optical microscope, counting, and converting to virus titer through immunocytochemistry using an anti-hexon polyclonal antibody 48 hours after infecting HEK293 cells with 0, 5, 10, 25, or 50 MOI virus doses of HAdV-5 and chimeric adenoviruses prepared in the present invention. The X-axis represents the dose of virus infected cells, and the Y-axis represents the HAdV-5, ChimAd, and ChimAd-H28 virus titers according to each virus dose. * indicates the significance level between the HAdV-5 virus and the chimeric adenovirus verified using a paired t-test (* p<0.05, ** p<0.01),
Figure 13 shows the in vitro neutralizing antibody test in HEK293 cells by reacting HAdV-5 and chimeric adenoviruses prepared in the present invention with anti-HAdV-5 polyclonal antibodies, and then measuring the number of adenovirus-infected cells and the measured values are plotted graphically. The X axis represents the dilution factor of the anti-HAdV-5 polyclonal antibody serially diluted from 10¹ to 10⁴, and the Y axis represents the number of cells infected by HAdV-5, ChimAd and ChimAd-H28 viruses. * indicates the level of significance between the HAdV-5 virus and the chimeric adenovirus verified using a paired t-test (* p<0.05, ** p<0.01, *** p<0.005, **** p<0.0005),

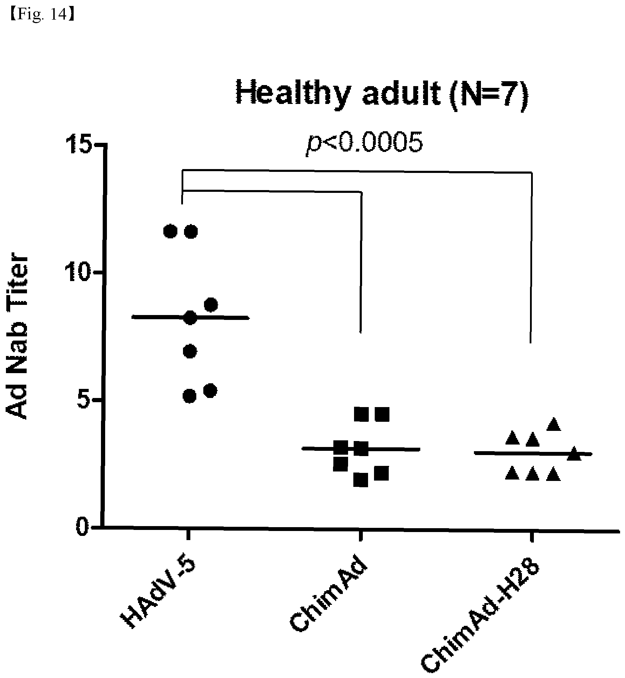

Figure 14 shows the result of in vitro neutralizing antibody test in HEK293 cells by reacting HAdV-5 and chimeric adenoviruses prepared in the present invention with healthy human serum (n = 7) and the values expressed as reciprocal numbers by measuring the number of cells infected with adenovirus shown as a dot plot. The X-axis represents the adenovirus reacted with the serum, and the Y-axis represents the adenovirus neutralizing antibody (Ad Nab) titer for 7 human serum samples. The significance level between the HAdV-5 virus, the ChimAd virus, and the ChimAd-H28 virus, verified using a paired t-test, is shown as a P value,
Figure 15 is a schematic diagram of cloning ChimAd-CV and ChimAd-CVN using the Gibson assembly gene synthesis method in a ChimAd vector obtained through gene synthesis,
Figure 16 shows the SARS-Coronavirus-2 S protein results(A) and beta variant S protein results(B) for virus-binding antibody titers induced by ChimAd-CV and ChimAd-CVN using BALB/c female mice,
Figure 17 shows the neutralizing antibody titers induced by ChimAd-CV and ChimAd-CVN in BALB/c female mice as neutralizing antibody measurement results (A, B) using a beta variant (B.1.351 variant type) pseudovirus.
Figure 18 shows the results of cellular immunization (A, B) using S peptides of ChimAd-CV and ChimAd-CVN as stimulating antigens and the results of cellular immunization (C, D) using NP peptides as stimulating antigens, using BALB/c female mice,
Figure 19 shows the results of S protein of SARS-coronavirus-2(A) and the S protein of delta variant strain(B) induced by ChimAd-CV and ChimAd-CVN using hACE2 transgenic female mice,
Figure 20 shows the results of neutralizing antibody measurement using FRNT of SARS-coronavirus-2 (A, B) and of the delta variant strain(C,D) as a result of confirming the viral neutralizing antibody titer induced by ChimAd-CV and ChimAd-CVN using hACE2 transgenic female mice,
Figure 21 shows survival rate (A, B) and the average body weight (C,D) after SARS-coronavirus-2 (Wuhan-Hu-1), delta variant (B.1.617.2) challenge test in mouse animal models immunized by ChimAd-CV and ChimAd-CVN using hACE2 transgenic female mice,
Figure 22 shows coronavirus titers detected in the lungs after the challenge test,
Figure 23 is a schematic diagram of cloning ChimAd-HPV by using the Gibson assembly gene synthesis method in a ChimAd vector from a human papillomavirus gene obtained through gene synthesis,
Figure 24 shows the results of cellular immunization (A, B) using the HPV16-type E7 peptide as a stimulating antigen, as a result of ELISPOT, showing the specific T cell immune response to the ChimAd-HPV vaccine candidate.
Figure 25 shows the results of measuring the size of tumors, after subcutaneously injecting TC-1 cells, a tumor cell line, into C57BL/6 mice, and vaccine candidates, including a control group were injected before and after tumor cell injection to measure the size of cancer over time Results showing the lowest tumor volume in the ChimAd-HPV group (A, B) are shown.

### [Mode for Invention]

Hereinafter, the present invention will be described in more detail by the following examples. However, the following examples are described with the intention of illustrating the present invention, and the scope of the present invention is not to be construed as being limited by the following examples.

### [Example]

### Example 1: Gene synthesis for construction of chimeric adenoviral vectors

### 1-1) Synthetizing ChAdV-6 knob gene

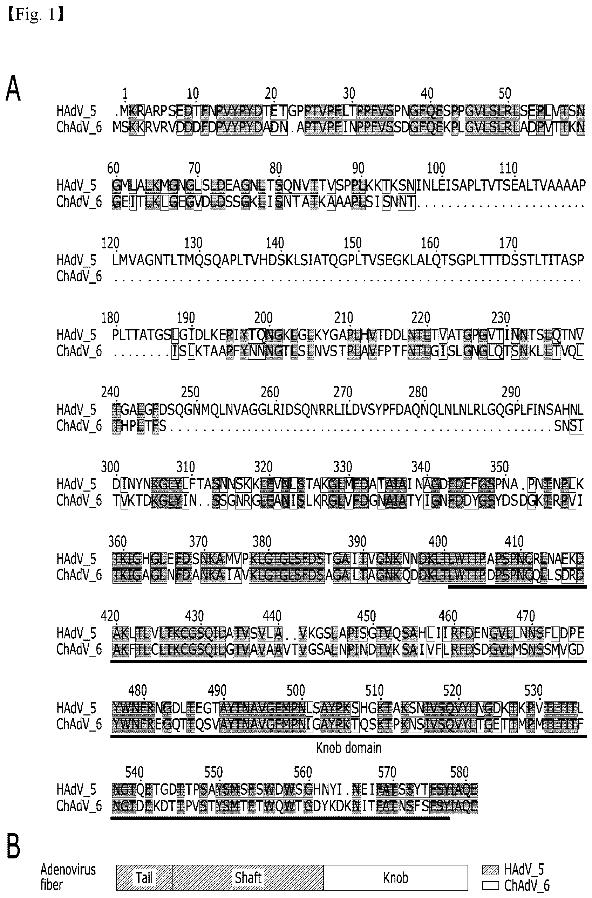

In order to construct a chimeric adenoviral vector by replacing the knob domain at the end of the fiber protein of HAdV-5 with the corresponding gene of ChAdV-6, a non-human adenovirus, genetic homology of the amino acid sequence to the fiber protein of HAdV-5 (Genebank: AY339865.1) and ChAdV-6 (Genebank : AY530877) was analyzed using the Clustal Omega program. Finally, the Clustal Omega results visualized the homology region through ESPript figure (FIG. 1A), and among the knob domains of ChAdV-6 (SEQ ID NO: 1), a base sequence corresponding to 405 to 574 amino acids (SEQ ID NO: 1), which is a region with low homology to the gene sequence of HAdV-5, was obtained by gene synthesis. (FIG. 1B, SEQ ID NO: 2).

### 1-2) Synthetizing HAdV-28 hypervariable region (HVR) gene

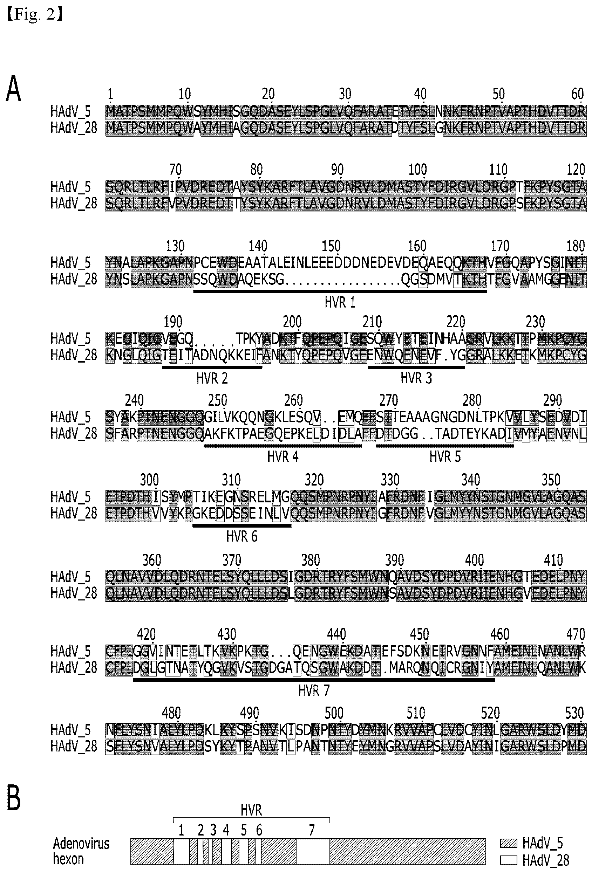

To construct a chimeric adenovirus vector by replacing the hypervariable region in the hexon protein of HAdV-5 with the corresponding gene of HAdV-28, an adenovirus serotype with low seroprevalence in humans, genetic homology of the amino acid sequence to the hexon protein of HAdV-5 (Genebank: AY339865.1) and HAdV-28 (Genebank: DQ149626.1) were analyzed using the Clustal Omega program. Finally, the Clustal Omega results were visualized through the ESPript figure, and the HVR1 region to the HVR7 region with low genetic homology of amino acid sequences between HAdV-5 and HAdV-28 were set (Table 1, Fig. 2A). Subsequently, to replace the HVR region in the hexon protein of HAdV-5 with the corresponding gene of HAdV-28, the nucleotide sequence from the beginning of the HVR1 region of HAdV-28 (132 amino acids) to the end of the HVR7 region (449 amino acids) was gene synthesized. (FIG. 2B, SEQ ID NO: 4). the genetic homology of amino acid sequences between HAdV-5 and HAdV-28 was set from the HVR1 region to the HVR7 region (Table 1, Fig. 2A). Subsequently, to replace the HVR region in the hexon protein of HAdV-5 with the corresponding gene of HAdV-28, the nucleotide sequence from the beginning of the HVR1 region of HAdV-28 to the end of the HVR7 region was gene synthesized. (FIG. 2B, SEQ ID NO: 4).

**[TABLE 1]**

| **Target** | **HVR region** | **Position** | **Sequence** |
|---|---|---|---|
| HAdV-5 | HVR1 | 132-168 | PCEWDEAATALEINLEEEDDDNEDEVDEQAEQQKT HV (SEQ ID NO: 15) |
| | HVR2 | 188-195 | VEGQTPKY (SEQ ID NO: 16) |
| | HVR3 | 209-220 | SQWYETEINHAA (SEQ ID NO: 17) |
| | HVR4 | 248-265 | GILVKQQNGKLESQVEMQ (SEQ ID NO: 18) |
| | HVR5 | 268-284 | STTEATAGNGDNLTPKV (SEQ ID NO: 19) |
| | HVR6 | 305-316 | TIKEGNSRELMG (SEQ ID NO: 20) |
| | HVR7 | 418-458 | GGVINTETLTKVKPKTGQENGWEKDATEFSDKNEI RVGNNF (SEQ ID NO: 21) |
| HAdV-28 | HVR1 | 132-153 | SSQWDAQEKSGQGSDMVTKTHT (SEQ ID NO: 22) |
| | HVR2 | 173-185 | TEITADNQKKEIF (SEQ ID NO: 23) |
| | HVR3 | 199-209 | ENWQENEVFYG (SEQ ID NO: 24) |
| | HVR4 | 237-256 | AKFKTPAEGQEPKELDIDLA (SEQ ID NO: 25) |
| | HVR5 | 259-273 | DTDGGTADTEYKADI (SEQ ID NO: 26) |
| | HVR6 | 294-305 | GKEDDSSEINLV (SEQ ID NO: 27) |
| | HVR7 | 407-449 | DGLGTNATYQGVKVSTGDGATQSGWAKDDTMAR QNQICRGNIY (SEQ ID NO: 28) |

Table 1 shows HVR regions selected through nucleotide sequence analysis between HAdV-5 and HAdV-28.

### 1-3) Synthesis of vector regulators

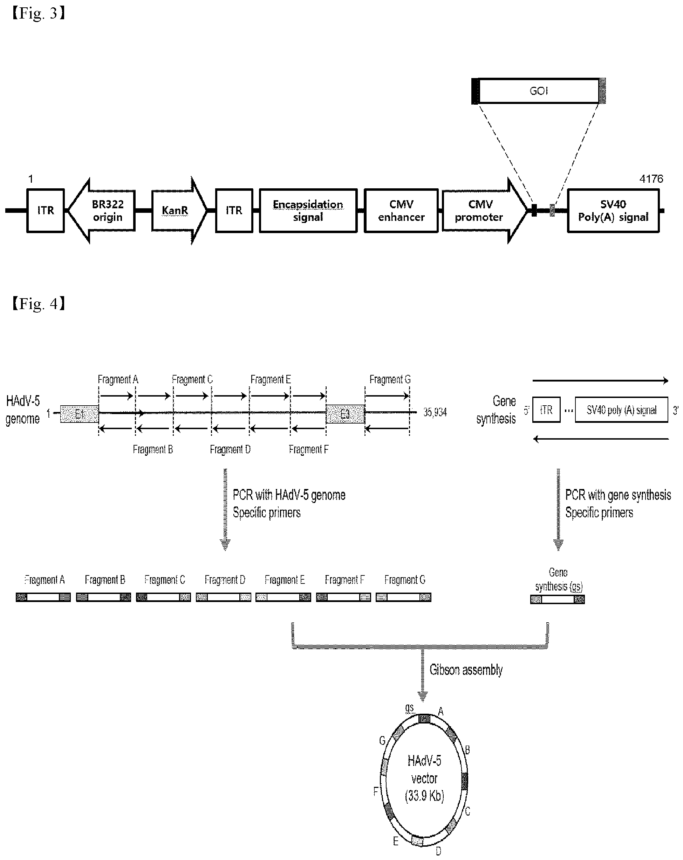

Vector regulators to be inserted into the chimeric adenovirus vector were synthesized by combining vector regulators known to date (e.g., promoter, origin of replication, etc.). In more detail, corresponding vector regulators were obtained by synthesizing genes in the order of ITR (inverted terminal repeat, Genebank: AY339865.1), BR322 origin of replication (Genebank: MT612434.1), kanamycin resistance gene (Genebank: MT084773.1), ITR sequence, encapsidation signal, Genebank: AY339865.1), CMV enhancer (Genebank: MT267310.1), CMV promoter (Genebank: MT267310.1), and SV40 poly(A) (Genebank: MT612432.1) (FIG. 3).

### Example 2: Preparation of chimeric adenoviral vectors

### 2-1) Preparing HAdV-5 vector

Using DNA of HAdV-5 (ATCC, VR-1516) as a template, PCR amplification was performed using primers except for some of the E1 and E3 genes to obtain the amplified products (fragments A-G) (Table 2). The synthetic gene (4.1 Kb) comprising the 7 vector PCR amplification products and the synthesized CMV promoter sequence has the same sequence (about 20 bp) at each end, and were combined through a gibson assembly (NEB, E2611S) reaction to construct HAdV- 5 vectors (FIG. 4). A ChimAd vector was constructed using this as a basic vector.

**[TABLE 2]**

| **Primer name** | **Sequence (5' to 3' direction)** | **PCR produc t** |
|---|---|---|
| Fragment A | F: TAAGGGTGGGAAAGAATATATAAGGTGGGGGTC (SEQ ID NO: 29) | 4 Kb |
| | R: GCC TTC CAT CAA GGG CAT CCC G (SEQ ID NO: 30) | |
| Fragment B | F: GTAAAGTTCCAAGAAGCGCGGGATGC (SEQ ID NO: 31) | 4 Kb |
| | R: AGTTAATCTCCTGGTTCACCGTCTG (SEQ ID NO: 32) | |
| Fragment C | F: GTAACCGCATACGAGCAGACGGTG (SEQ ID NO: 33) | 4 Kb |
| | R: CGTGGAGCGGAAGGTCACG (SEQ ID NO: 34) | |
| Fragment D | F: GCCAGACATGATGCAAGACCCCGTG (SEQ ID NO: 35) | 4 Kb |
| | R: CGTACCACTGAGATTCTCCTATTTGAGGTTC (SEQ ID NO: 36) | |
| Fragment E | F: CAACCTGAACCTCAAATAGGAGAATCTCAG (SEQ ID NO: 37) | 4 Kb |
| | R: GGATAACGATCTAAAGGCGAACTTCAAAC (SEQ ID NO: 38) | |
| Fragment F | F: GTCAGGCAGTAGTTTGAAGTTCGC (SEQ ID NO: 39) | 4.7 Kb |
| | R: CTCTAGTTATAACTAGAGGATCTTGATGTAATCCAGGGTTAG (SEQ ID NO: 40) | |
| Fragment G | F: CCTCTAGTTATAACTAGAGTACCCGGGATCTTATTCCCTTTA AC (SEQ ID NO: 41) | 5 Kb |
| | R: CGGAGTAACTTGTATGTGTTGGGAATTG (SEQ ID NO: 42) | |
| Gene synthesis (promoter, etc.) | F: CAATTCCCAACACATACAAGTTACTCCG (SEQ ID NO: 43) | 5 Kb |
| | R: TATATTCTTTCCCACCCTTACGCGTTAAGATACATTGATG (SEQ ID NO: 44) | |

Table 2 shows PCR primers used to construct HAdV-5 vector

### 2-2) Preparation of ChimAd vector

A vector PCR amplification product was obtained by PCR amplification using the HAdV-5 vector prepared above as a template and using the primers shown in Table 3, excluding the knob gene sequence. The synthesized ChAdV-6 knob gene was PCR amplified to have the same sequence (about 20 bp) as the vector PCR amplification product at the 3' and 5' ends to obtain an insert gene, and was combined with the vector PCR amplification product through gibson assembly reaction to construct a ChimAd vector (Figs. 5 and 6).

**[TABLE 3]**

| **Primer name** | **Sequence (5' to 3' direction)** | **PCR product** |
|---|---|---|
| 6Knob vector | F: CCTTTGCTACCAACTCATTCTCTTTTTCATACATTGCCC (SEQ ID NO: 45) | 33.4 Kb |
| | R: ACAATTAGGGCTTGGGTCAGGTGTGGTCCACAAAGTTAG C (SEQ ID NO: 46) | |
| 6Knob insert | F: GCTAACTTTGTGGACCACACCTGACCCAAGCCCTAATTGT (SEQ ID NO: 47) | 0.5 Kb |
| | R: GGGCAATGTATGAAAAAGAGAATGAGTTGGTAGCAAAGG (SEQ ID NO: 48) | |

Table 3 shows the PCR primers used to amplify the vector and the knob gene for the construction of the ChimAd vector

### 2-3) Preparation of the ChimAd-H28 vector

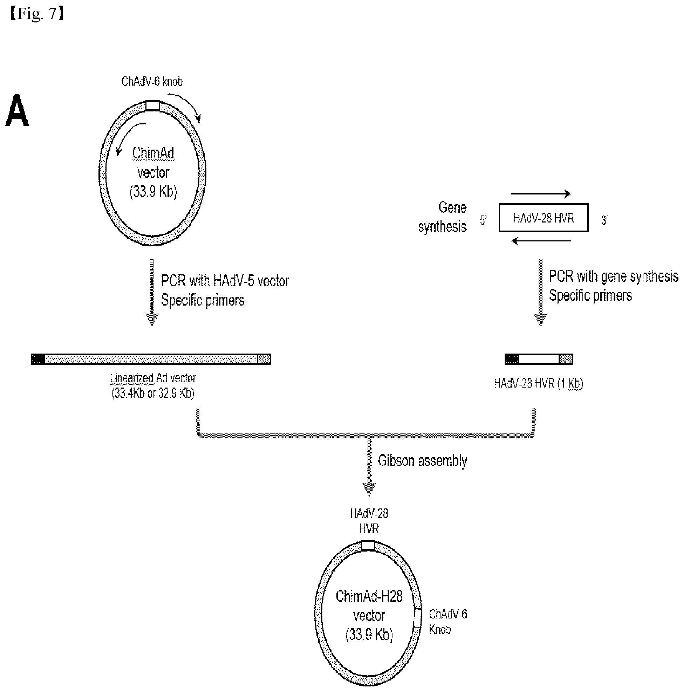

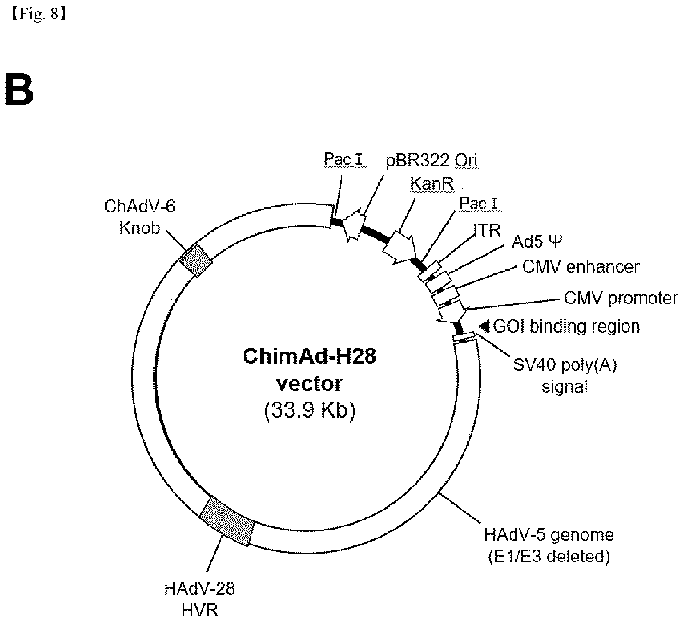

Using the ChimAd vector as a template, the vector sequence excluding the HVR region (19,879-20,858 bp) of HAdV-5 was PCR amplified using the primers in Table 4 by PCR, and the amplified product (32.9 Kb) and the PCR amplified product of the HVR sequences of HAdV-28 (1 Kb) synthesized above were combined through Gibson assembly reaction to construct a ChimAd-H28 vector (FIGS. 7 and 8).

**[TABLE 4]**

| **Primer name** | **Sequence (5' to 3' direction)** | **PCR product** |
|---|---|---|
| 28HVR vector | F: TGCAGGGGTAACATCTACGCCATGGAAATCAATCTAAAT G (SEQ ID NO: 49) | 32.9 Kb |
| | R: TGCGCATCCCACTGACTGGAATTTGGGGCACCCTTGGGAG (SEQ ID NO: 50) | |
| 28HVR insert | F: CTCCCAAGGGTGCCCCAAATTCCAGTCAGTGGGATGCGC A (SEQ ID NO: 51) | 1 Kb |
| | R: CATTTAGATTGATTTCCATGGCGTAGATGTTACCCCTGCA (SEQ ID NO: 52) | |

Table 4 shows the PCR primers used to amplify the vector and the 28-type hexon HVR gene for the construction of the ChimAd-H28 vector.

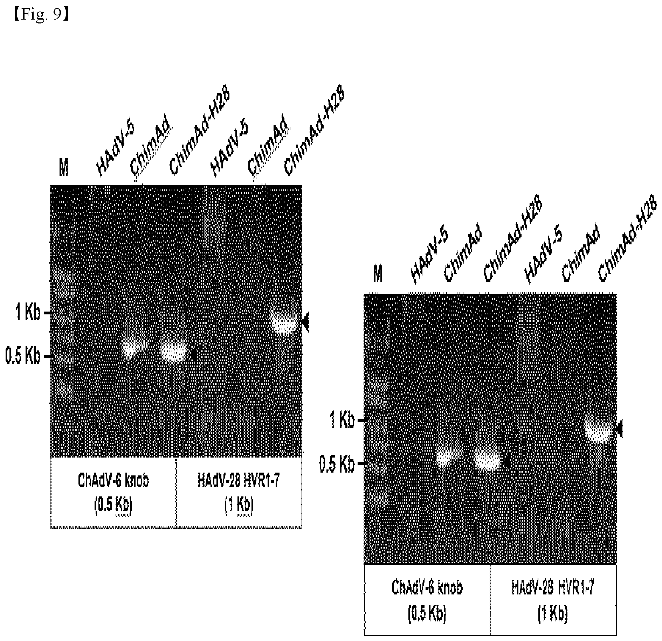

As shown in Figure 9, only the ChAdV-6 knob gene inserted in 0.5 Kb is amplified in the ChimAd vector by the PCR method, In the ChimAd-H28 vector using this as a template, the amplification of the ChAdV-6 knob gene and the 1 Kb HAdV-28 hexon HVR gene were simultaneously confirmed. Through this, two final chimeric adenoviral vectors (ChimAd, ChimAd-H28) were obtained by confirming the presence or absence of the chimeric capsid gene in the HAdV-5 vector.

### Example 3: Production of adenovirus

To evaluate the virus formation of HAdV-5 and the two chimeric adenoviruses designed and produced in the present invention, each vector was cut and removed from the pBR322 replication start point and kanamycin resistance gene sequence unnecessary for adenovirus formation using Pac I restriction enzyme, and HEK293 cells (human embryonic kidney; ATCC, CRL-1573) expressing E1 and E3 genes subcultured the previous day at 1.6 × 10⁶ cells in a 100 mm cell culture dish were transformed using Lipofectamine 2000 (Invitrogen, #11668027).

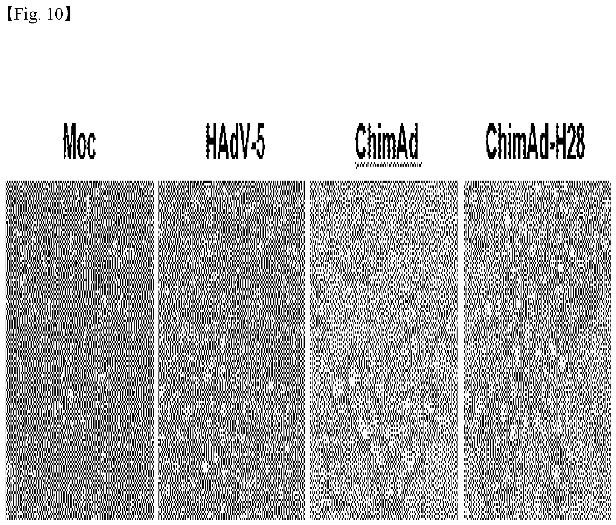

Transformed cells were cultured for 10-20 days in an incubator maintained at 37°Cand 5.0% CO₂ partial pressure, and the formation of a cytopathic effect by adenovirus infection was monitored (FIG. 10). To harvest HAdV-5 and two chimeric adenoviruses from whole CPE, the cells attached to the bottom of the flask were detached using a scraper, and the supernatant was removed by centrifugation at 4 °C and 500 x g together with the culture medium, and after washing with 1X PBS, adenovirus was released by repeating the freezing-thawing process three times in the resulting pellet. Cell debris was centrifuged at 14,000 x g at room temperature, and the adenovirus-containing supernatant was dispensed into 1.5 mL microcentrifuge tubes and stored at -80°C

As shown in FIG. 10, the formation of CPE by transformation in HEK293 cells using the HAdV-5, ChimAd, and ChimAd-H28 vectors prepared in the present invention was confirmed at 200x magnification through an optical microscope. From these results, it is considered that adenovirus replication was achieved in the host cell by the three vectors constructed above, and cytotoxicity was induced by the virus propagation or the expressed adenovirus protein.

### Example 4: Western blot analysis for confirming adenovirus envelope protein

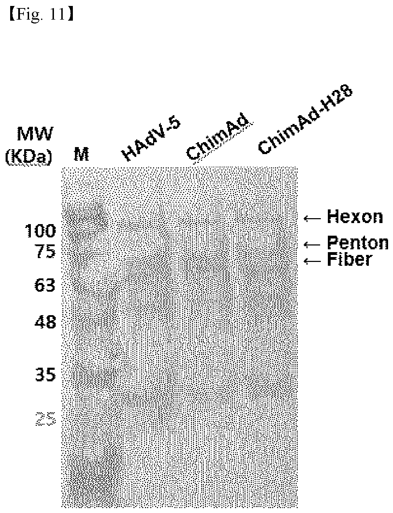

To confirm the expression of adenoviral structural proteins for HAdV-5 and chimeric adenoviruses produced in the HEK293 cell line, Western blot analysis was performed as follows. After adding SDS-PAGE loading dye to HAdV-5, ChimAd, and ChimAd-H28 viruses and bathing at 100 for 15 minutes, and were separated by electrophoresis on 10% SDS-PAGE at 100 V, and then transferred to a PVDF membrane. After removing the activity of the PVDF membrane with 5% skim milk solution, the primary antibody, anti-HAdV-5 polyclonal antibody (Abcam, #ab6982) was diluted 1:1000 with TBS solution and reacted at room temperature for 1 hour. After washing three times with TBS-T buffer solution, the secondary antibody, HRP-attached goat-derived anti-mouse immunoglobulin G was diluted 1:5000 with TBS solution, reacted at room temperature for 1 hour, and then washed with TBS-T buffer solution repeatedly. Confirmation of Western results was confirmed after color development using diaminobenzidine (Sigma, #34002) (FIG. 11).

As shown in FIG. 11, in HAdV-5, ChimAd and ChimAd-H28 viruses, In the virus, hexon (about 108 kDa), fiber (about 61 kDa), and penton (about 68 kDa) proteins were detected in response to all anti-HAdV-5 polyclonal antibodies specifically. When comparing the detected fiber protein of the HAdV-5 virus and the detected fiber protein of the ChimAd virus, since fiber proteins of the same size were confirmed in ChimAd virus, it can be seen that the chimAd virus knob protein replaced with the ChAdV-6 knob gene was normally expressed. In addition, hexon protein of the same size was confirmed in HAdV-5 and ChimAd-H28, indicating that the hexon protein of the ChimAd-H28 virus replaced with the HVR gene of HAdV-28 was normally expressed. In addition, it can be confirmed that the main coat proteins (hexon, fiber, and penton) of adenovirus are stably expressed by the two chimeric adenovirus vectors.

### Example 5: Analysis of adenovirus infectivity in HEK293 cells

To analyze the cell infectivity of two chimeric adenoviruses in which the knob protein sequence that binds to the CAR receptor of the host cell and acts on cell entry is replaced with the gene sequence of ChAdV-6, cells infected with HAdV-5, ChimAd and ChimAd-H28 viruses were observed under the same MOI virus dose condition.

Adenovirus infection titer was measured using the QuickTiter^{™} titer immunoassay kit (Cell biolabs, #VPK-109) of immunocytochemistry using an anti-hexon polyclonal antibody that binds to adenovirus hexon protein. The adenovirus to be used for titer measurement was diluted from 10¹ to 10⁶ by 10-fold serial dilution using 1X PBS, and infection was induced by dispensing 100 µl of adenovirus dilution into HEK293 cells in a 24-well plate (SPL, #30024) prepared with a cell count of 2.5 × 10⁵ by subculture 1 hour before. Immunostaining for detection of adenovirus was performed 48 hours after infection in an incubator maintained at a temperature of 37 and a partial pressure of CO₂ of 5.0%. Cells infected for 48 hours were fixed by removing the supernatant, dispensing cold methanol, and leaving the cells at -20°C for 20 minutes. After sufficiently removing methanol from the fixed cells with 1X PBS solution, the activity was inhibited with 1% skim milk (Difco, #232100) solution, and the primary antibody, anti-hexon antibody, was diluted with 1XPBS and reacted at room temperature for 1 hour. After washing three times with 1X PBS, the HRP-conjugated secondary antibody was diluted to 1X and reacted. Then, using 3.3'-diaminobenzidine stain kit (DAB) (Thermo Fisher, #34002), color was developed for 10 minutes, and then, using an optical microscope, after select the virus dilution interval in which brown spots, which are positive cells infected with adenovirus, are observed between 50 and 100, the infection titer (pfu/mL) of HAdV-5, ChimAd and ChimAd-H28 viruses was measured by applying the adenovirus titer conversion formula provided by Cell Biolabs.

For the infectivity analysis, HEK293 cells subcultured in a 24-well plate at 2.5×10⁵ cells were incubated for 1 hour in an incubator at a temperature of 37 and a CO₂ partial pressure of 5.0%, and then based on the pfu/mL value obtained from the virus titer measurement, HAdV-5 and chimeric adenoviruses were diluted with 1X PBS at 0, 5, 10, 25, and 50 MOI virus doses, respectively, to induce infection in the cells. 48 hours after infection, the number of adenovirus-infected cells at each MOI virus dose was counted in at least 4 fields at 200X magnification under an optical microscope through immunocytochemical staining using an anti-hexonpolyclonal antibody (Table 5), and the number of positive cells (brown spots) was converted into pfu/mL values, and the infectivity of HAdV-5 and chimeric adenoviruses for each MOI virus dose was compared (FIG. 12).

**[TABLE 5]**

| **Ad infected cell spots** | **Virus dose (MOI)** | | | |
|---|---|---|---|---|
| | 5 | 10 | 25 | 50 |
| HAdV-5 | 4.0 | 8.1 | 16.6 | 22.2 |
| ChimAd | 10.1 | 17.9 | 21.6 | 34.6 |
| ChimAd-H28 | 9.5 | 17.7 | 19.8 | 32.3 |

Table 5 shows the number of cells infected by HAdV-5 and chimeric adenovirus at the same MOI. As shown in Table 5, when HEK293 cells were infected with the same time and MOI virus dose, the number of cells infected with ChimAd and ChimAd-H28 viruses was higher than that of HAdV-5 virus.

In addition, when comparing the virus titers for each MOI virus dose in FIG. 12, the ChimAd virus and ChimAd-H28 virus had 1.3-3 times and 1-2 times more virus titers than HAdV-5 virus, respectively, showed a scientifically significant difference. Through these results, it was confirmed that not only cell infectivity but also virus productivity was increased due to replacement of the ChAdV-6 knob gene and the HAdV-28 hexon HVR gene.

### Example 6: In vitro neutralizing antibody test using anti-HAdV-5 polyclonal antibody

To evaluate the existing immune evasion ability of the HAdV-5 vector due to replacement of the knob gene and HVR region of hexon, which is the main neutralizing antibody recognition site, in the two chimeric adenoviruses produced, an in vitro neutralizing antibody test was performed using an anti-HAdV-5 polyclonal antibody (Abcam, #ab6982) as a neutralizing antibody against HAdV-5.

In the in vitro neutralizing antibody test, the anti-HAdV-5 antibody was diluted using 1X PBS solution to a 10-4 dilution factor through serial dilution, and after neutralizing with 1.25 × 10⁷ VP of HAdV-5, ChimAd, and ChimAd-H28 viruses corresponding to a 50 MOI virus dose for 1 hour at room temperature, 2.5 × 10⁵ cells were dispensed into HEK293 cells in a prepared 24-well cell culture plate, respectively and cultured in an incubator maintained at a temperature of 37 and a CO₂ partial pressure of 5.0%, and viral infection was induced for 48 hours. 48 hours after infection, immunocytochemical staining using an anti-hexon polyclonal antibody was performed, and by counting the number of positive cells infected with HAdV-5, ChimAd and ChimAd-H28 viruses in at least 4 fields at 200x magnification using an optical microscope for each anti-HAdV-5 polyclonal antibody dilution factor (10¹-10⁴), the ability to avoid neutralization against anti-HAdV-5 antibodies was analyzed (Table 6).

**[TABLE 6]**

| **Ad infected cell spots** | **Dilution of anti-HAdV-5 antibody** | | | |
|---|---|---|---|---|
| | 10¹ | 10² | 10³ | 10⁴ |
| HAdV-5 | 1.0 | 12.5 | 44.1 | 57.8 |
| ChimAd | 4.4 | 40.1 | 97.0 | 100.8 |
| ChimAd-H28 | 2.6 | 13.5 | 69.5 | 100.1 |

Table 6 shows the number of cells infected with HAdV-5 and chimeric adenovirus after reaction with anti-HAdV-5 polyclonal antibody. As shown in FIG. 13, compared to the HAdV-5 virus, by ChimAd virus about 3.1 to about 4.5 times at low antibody dilution factor (10¹ - 10²) and about 1.8 to about 2.2 times more adenovirus-infected cells at high antibody dilution factor (10³ to 10⁴). ChimAd-H28 virus also confirmed about 2.8 times more adenovirus-infected cells than HAdV-5 virus at a low antibody dilution factor and about 1.5 to about 1.7 times at a high antibody dilution factor, indicating a statistically significant difference. Through this, it was obtained that the neutralizing antibody reaction can be avoided up to 4.5 times only by replacing the knob gene in the adenovirus capsid protein. In addition, the above results are 2 times higher avoidance activity than the result of in vitro neutralizing antibody test of a chimeric adenoviral vector (2 fold avoidance compared to HAdV-5 vector) in which the recently developed HAdV-5 structure was replaced with the HAdV-3 knob gene.

As a result, the ChimAd vector constructed in the present invention is considered to be an adenoviral vector with superior immune evasion ability against HAdV-5 than conventional chimeric adenoviral vectors.

### Example 7: In vitro neutralizing antibody test using human serum

To evaluate the neutralizing antibody titer in human serum capable of recognizing ChimAd and ChimAd-H28 viruses, which are the chimeric adenoviral vectors constructed in the present invention, an in vitro neutralizing antibody test was conducted using the serum of 7 healthy adults. The complement cascade was inactivated in human serum by heat-inactivation at 56 for 30 minutes, and then serially diluted two-fold with 1X PBS. Thereafter, the inactivated sera were neutralized with adenovirus under the same 50 MOI virus dose condition at room temperature for 1 hour, and then through the in vitro neutralizing antibody test method described in Example 6, the number of positive cells (brown spots) was measured in at least four fields at 200x magnification using an optical microscope and expressed as an average to determine whether HAdV-5 and chimeric adenoviruses were infected in HEK293 cells on day 2 of infection (Table 7). The number of stained cells was converted into a reciprocal number and then multiplied by 100 to derive neutralizing antibody titers for HAdV-5, ChimAd, and ChimAd-H28 viruses in human serum, and expressed as a dot plot (FIG. 14).

**[TABLE 7]**

| **Ad infected cell spots** | **Human serum (N=7)** | | | | | | |
|---|---|---|---|---|---|---|---|
| | #1 | #2 | #3 | #4 | #5 | #6 | #7 |
| HAdV-5 | 18.5 | 14.4 | 1.4 | 8.6 | 19.25 | 11.4 | 8.6 |
| ChimAd | 45.2 | 50.8 | 23.2 | 22 | 39 | 31.2 | 22 |
| ChimAd-H28 | 44 | 44 | 6 | 27.2 | 43.6 | 27.6 | 23.8 |

Table 7 shows the number of cells infected by HAdV-5 and the chimeric adenovirus following reaction with human sera. As can be seen in Table 7, in the seven human serogroups, we identified cells infected on average 4.6 times more by ChimAd virus than by HAdV-5 virus and by average 2.9 times more cells infected by ChimAd-H28 virus than by HAdV-5 virus, which was a statistically significant difference. In addition, as can be seen in Figure 13, which shows the number of stained cells as the reciprocal number, it can be seen that the average 2.6-2.7 times lower Ad neutralizing antibody (NAb) titer in the ChimAd virus than in the HAdV-5 virus. These results indicate that vector immunity is lower when administering ChimAd vector-based or ChimAd-H28 vector-based vaccines to humans than HAdV-5 vector-based vaccines, so it will be an optimal adenoviral vector that can deliver foreign genes more efficiently.

### Example 8: Application of ChimAd vector-based corona vaccine

### 8-1) Genetic modification encoding SARS-CoV-2 S protein and N protein

To develop a vaccine using the coronavirus structural protein, two S polypeptide synthetic genes comprising NTD and CTD of Spike protein and N protein including modifications such as amino acid substitution were obtained.

Specifically, in the case of the CV gene, SARS-Coronavirus-2 of the beta-variant S protein was introduced with glycine at 614, acidic amino acid substitution of residues 682 to 685, and consecutive proline substitution mutations at 986 and 987. [Pallesen, Jesper, et al. Proceedings of the National Academy of Sciences 114.35 (2017): E7348-E7357. Bangaru, Sandhya, et al. Science 370.6520 (2020): 1089-1094. Plante, Jessica A., et al. Nature 592.7852 (2021): 116-121.].

In the case of the CVN gene, positions 44 to 180 and 247 to 364 of the N protein were linked to the N-terminus and C-terminus of the Spike protein through a (GGGGS)₃ linker, respectively, and at this time, additional structure prediction was performed through prediction tools such as trRosetta and Alphafold. [Du, Zongyang, et al. Nature Protocols (2021): 1-18. Jumper, John, et al. Nature 596.7873 (2021): 583-589.]

### 8-2) Gene synthesis to be used as SARS-CoV-2 vaccine material

To develop a vaccine using coronavirus structural proteins, synthetic genes encoding Spike and Nucleocapsid proteins among structural proteins were obtained. As shown in Table 8, for each vaccine candidate, S protein derived from coronavirus B.1.351 and N protein derived from Wuhan-Hu-1 were selected and codon optimized for easy expression in adenovirus. The region comprising the S protein was named CV, and the chimera form in which a portion of the N protein and the S protein were fused was named CVN.

**[TABLE 8]**

| | **Region** | **Accession no.** |
|---|---|---|
| CV | Spike surface glycoprotein | QUT64557(B. 1.351) |
| CVN | NP(NTD)-Spike-NP(CTD) | QUT64557(B.1.351), MN908947(Wuhan-Hu-1) |

### 8-3) Cloning chimeric adenovirus comprising SARS-CoV-2 gene

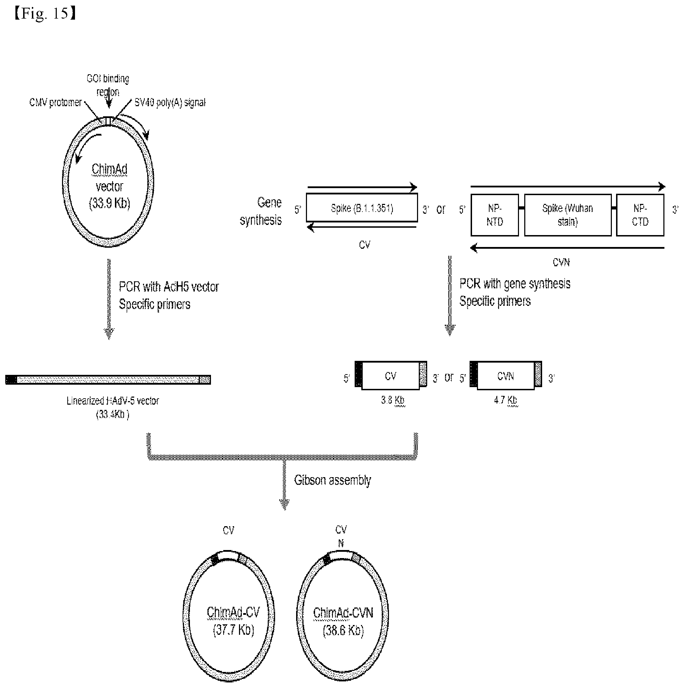

In order to clone the two SARS-CoV-2 vaccine antigens synthesized above into the ChimAd vector, which is a chimeric adenovirus vector, using the plasmid DNA of the ChimAd vector as a template, PCR amplification was performed except for a part of the GOI binding region gene, and the amplified product (about 33.4 Kb) was purified through gel extraction. The insertion gene for cloning was PCR amplified to comprise the same sequence (homologous region, about 20 bp) as the 5' and 3' ends of the GOI binding region in the ChimAd vector, and the amplification product CV (about 3.8 Kb) and CVN (about 4.7 Kb) were obtained, and then combined with the previously purified DNA PCR amplification products through a gibson assembly reaction, respectively, to finally obtain two types of ChimAd-CV and ChimAd-CVN plasmids (FIG. 15).

**[TABLE 9]**

| **Primer name** | **Sequence (5' to 3' direction)** | **PCR product** |
|---|---|---|
| ChimAd vector | F: CTAAGCTTCTAGATAAGATATCCGATCCAC (SEQ ID NO: 53) | 33.4 Kb |
| | R: CGCGTCGACGGTACCAGATCTCTAGCGGATC (SEQ ID NO: 54) | |
| CV region | F: GATCTGGTACCGTCGACGCGATGTTTGTGTTTCTGGTGCTG (SEQ ID NO: 55) | 3.8 Kb |
| | R: TATCTTATCTAGAAGCTTAGTCAGGTATAGTGCAGTTTCAC (SEQ ID NO: 56) | |
| CVN region | F: GATCTGGTACCGTCGACGCGATGGGCCTGCCCAACAACAC (SEQ ID NO: 57) | 4.7 Kb |
| | R: TATCTTATCTAGAAGCTTAGTCAGGGGAAGGTCTTGTAGGC (SEQ ID NO: 58) | |

### 8-4) Production and purification of chimeric adenovirus-based corona vaccine candidate

To produce a chimeric adenovirus-based coronavirus vaccine candidate using the ChimAd-CV and ChimAd-CVN plasmids produced in the present invention, each plasmid was treated with Pac I restriction enzyme (NEB, R0547L) to cut and remove the pBR322 replication start point and kanamycin resistance gene sequence that are unnecessary for adenovirus formation, and HEK293 cells were transformed with Lipofectamine 2000 (Invitrogen, 11668027) and cultured for 10-20 days in an incubator at 37 and CO₂ partial pressure of 5.0% to obtain a primary virus stock.

ChimAd-CV and ChimAd-CVN virus production was performed by infecting HEK293 cells at a cell density of about 1x 10⁶ cells/ml with a titer of 2 MOI using the primary virus stock obtained above, and after 48 hours, 0.45 CFF microfiltration filter (Cytiva, CFP -4-E-6A) was used to enrich the cells. The recovered cells were treated with 0.5% Tween20 (Sigma-aldrich, P2287-500ML) and 20 U/ml benzonase (Milipore, E1014-25KU) for 4 hours to induce cell lysis and host cell DNA degradation. Thereafter, cell debris was removed using 2 and 0.6 ULTA GF filters (Cytiva, KGF-A-0210TT, KGF-A-9606TT), and virus concentration and buffer exchange were performed through tangential flow filtration (TFF). ChimAd-CV and ChimAd-CVN virus were isolated from virus samples in PBS buffer through anion exchange chromatography using a HiTrap Capto Q ImpRes column (Cytiva, 17547051) and fast protein liquid chromatography (FPLC) using a Capto core 700 column (Cytiva, 17548115), and formulated into a vaccine buffer, and finally filtered with a 0.2 µm LTLTA GF filter (Cytiva, KGF-A-9610TT) to obtain two vaccine candidates.

### 8-5) Immunogenicity test results through binding antibody titer/neutralizing antibody titer/cellular immunity analysis

### (1) Immune induction using BALB/c female

Mice were immunized using the ChimAd-CV and ChimAd-CVN vaccine candidates obtained by the above manufacturing method. The test group includes a total of 4 groups: buffer, ChimAd used as a mock-up, ChimAd-CV, and ChimAd-CVN. For the immune induction test, 5-week-old BALB/c female mice were used, and each adenovirus test group was injected at a concentration of 2.5*10^9 ifu/dose. At 4-week intervals, the first and second vaccinations were administered by intramuscular injection twice (days 0 and 28), and blood was collected 4 and 6 weeks after the first vaccination (days 27 and 42), serum was separated and the spleen was removed.

### (2) ELISA; Binding antibody titer of mouse anti-serum by ChimAd-CV and ChimAd-CVN

A binding antibody titer test was performed to confirm the presence of antibodies in the serum through a mouse immunity induction test. To perform the test, coronavirus SARS-coronavirus-2 and beta variant S protein were dispensed into microplates at a concentration of 1µg/ml, 100ng/well each, and coated at 4°Cfor more than 16 hours. After reaction, the plate was washed at least 4 times with 1XPBS comprising Tween 20(PBS-T) and 100µl of 0.5% casein was dispensed and reacted at 37°Cfor 1 hour. After washing with PBS-T, each serum was diluted 1: 100 and then serially diluted 3-fold to approximately 1×10⁶. After dispensing and reacting at 37°C for 1 hour, the mixture was washed with PBS-T, and HRP-conjugated Goat anti-mouse IgG HRP (Thermo) was diluted 1:5000 and reacted for 1 hour. After the reaction was completed, to proceed with color development, 50µl of TMB solution (Thermo) was dispensed and color development was performed for 15-20 minutes. Afterwards, the reaction was stopped by adding an equal amount of Stop solution (Thermo), and the absorbance was measured at a wavelength of 450nm.

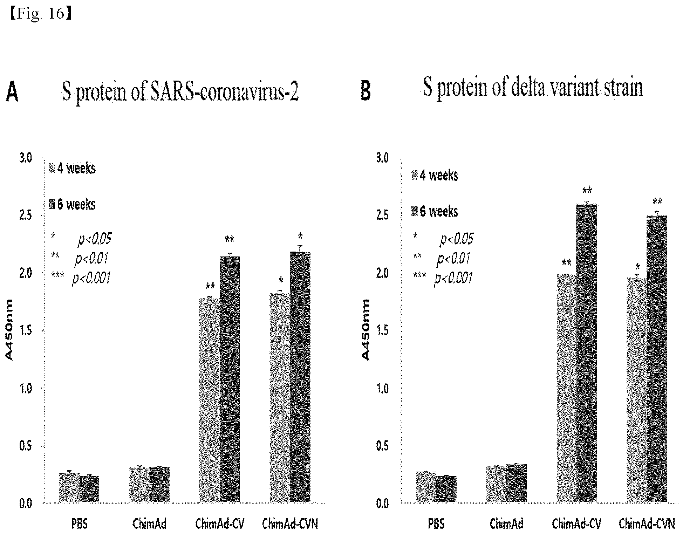

As a result of the binding antibody titer, it was confirmed that all experimental groups inoculated with ChimAd-CV and ChimAd-CVN showed high binding antibody titers of 10⁴ or more to SARS-coronavirus-2 and beta variant strains compared to the control group. In the experimental group inoculated with the control group, binding antibody titers to both SARS-coronavirus-2 and beta variant strains were measured, but the values were confirmed to be invalid (Figure 16).

### (3) pVNT (pseudovirus neutralization test)

; Measurement of neutralizing antibody titers against pseudoviruses in mouse anti-serum by ChimAd-CV and ChimAd-CVN

Neutralizing antibodies in serum were tested through a mouse immune induction test. The pseudovirus system is a virus that uses the backbone of Lentivirus to express the luciferase gene and the Spike protein of SARS-coronavirus-2 or beta variants. In this experiment, Takara's Lenti-X^{™} Packaging Mix product was used to produce pseudoviruses comprising the Spike protein of SARS-coronavirus-2 and beta variant strains. The produced pseudovirus was transformed into 293T cells overexpressing the ACE2 protein, luciferase expression was quantified and measured, and a neutralizing antibody test was performed using a certain RLU (Relative Light Units) value. For the neutralizing antibody test, each mouse serum was first inactivated at 56°Cfor 30 minutes, and DMEM medium comprising 10% FBS was used. The serum was diluted by 1/8 and then serially diluted 2-fold to about 10³. Pseudoviruses with a certain RLU value were mixed with the same amount of diluted serum and neutralization reaction was performed at 37°Cand 5% CO₂ incubator for 1 time. hACE2-293T cells cultured a day earlier were treated with 100µl of mixture of the virus and serum and cultured in an incubator at 37°Cand 5% CO₂ for about 72 hours. After completion of incubation, about 100µl of the culture medium was removed, 30µl of One-Glo luciferase reagent (Promega) was added, and reacted for 3-5 minutes. Then, 60µl of the mixture was transferred to a white plate and the luminescence value was measured using a luminometer. The measured RLU value was used to calculate the IC₅₀ value according to the serum dilution factor using a statistical program.

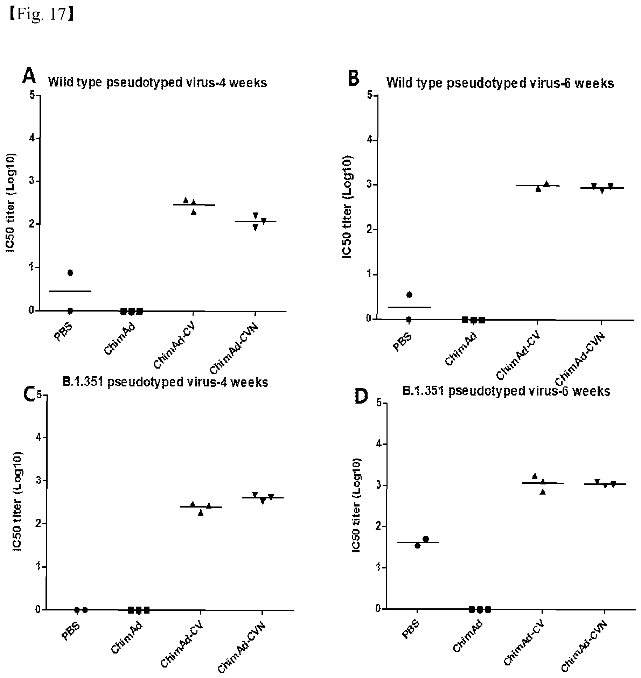

As shown in Table 10, the results of neutralizing antibodies against the beta variant virus showed IC₅₀ = 1253 for the ChimAd-CV inoculated group and IC₅₀ = 1131 for the ChimAd-CVN inoculated group, which were all higher than 10³, confirming a higher neutralizing antibody titer than the negative control group (Figure 17).

**[TABLE 10]**

| No | Group | pVNT(IC50) | |
|---|---|---|---|
| | | 4wk | 6wk |
| 1 | PBS | 7 | 42 |
| 2 | ChimAd | 9 | 0 |
| 3 | ChimAd-CV | 244 | 1253 |
| 4 | ChimAd-CVN | 409 | 1131 |

### (4)ELISpot; Specific T cell immune response caused by ChimAd-CV, ChimAd-CVN adenovirus

In the mouse immunity induction test, spleens were removed from each group 4 weeks after the first vaccination and 2 weeks after the second vaccination (6 weeks after the first vaccination). Spleen cells were recovered using Cell strainer (Falcon) and washed with PBS buffer solution. After washing, red blood cells were removed using red blood cell lysis buffer (Sigma), and the number of spleen cells was counted and diluted to the cell concentration used in the test. This experiment used R&D system's mouse IFN-gamma ELISpot kit, and the test was performed according to the manufacturer's instructions. For each test, 1×10⁵ cells, 2×10⁴ cells of spleen cells and Genscript's SARS-coronavirus-2 Spike protein peptide pool or Nucleprotein peptide pool were used as stimulating antigens and incubated together for 16 hours in a 37°C, 5% CO2 incubator. After incubation, the cells were washed with a washing solution, and 100 µl of biotin-conjugated IFN-gamma detection antibody was added and reacted by stirring at room temperature for 2 hours. After the reaction, the cells were washed four times using a washing solution, and 100 µl of streptavidin-AKP (alkaline phosphate) was added and incubated at room temperature for 2 hours. After the final washing, 100 µl of BCIP/NBT substrate was added and reacted at room temperature for about 45 minutes. When color development occurred due to the reaction, the spot was washed with sterilized water, the number of colored spots was counted, and the number of cells in the Spot Forming Unit (SFU) was calculated.

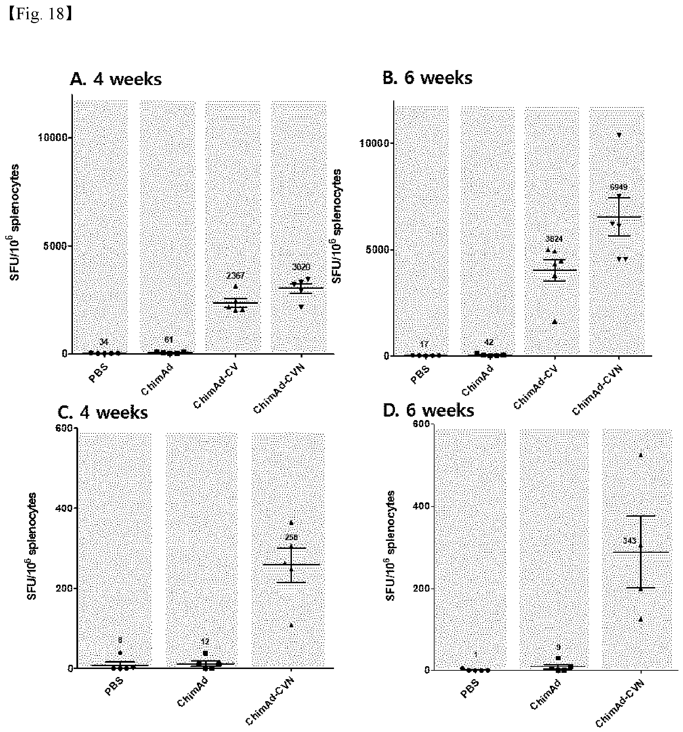

As a result of measuring the induction of specific T-cell immunity to each stimulating antigen of S protein and N protein through the ELISpot test, T-cell immunity was higher after the second vaccination (week 6) than after the first vaccination (week 4). Particularly it was confirmed that a specific immune response to the Spike protein peptide was induced in ChimAd-CVN, which was about two times higher than that in ChimAd-CV. In addition, it was confirmed that ChimAd-CVN induces a specific immune response to Nucleocapsid peptide (Figure 18).

**[TABLE 11]**

| No | Group | SPF/10^6 splenocytes (Spike peptide pool) | |
|---|---|---|---|
| | | 4wk | 6wk |
| 1 | PBS | 34 | 17 |
| 2 | ChimAd | 61 | 42 |
| 3 | ChimAd-CV | 2367 | 3824 |
| 4 | ChimAd-CVN | 3020 | 6949 |

### 8-6) Antibody titer, neutralizing antibody titer and challenge test after ChimAd-CV, ChimAd-CVN animal immunization

### A. Immune induction and challenge test using hACE2 transgenic female mouse

The immune induction and challenge tests conducted at IVI used 6-week-old female human ACE2 transgenic mice (Tg(K18-ACE2)2Prlmn) purchased from Jackson Laboratory (ME, USA). All the above tests were conducted in a BSL-3 level facility within the International Vaccine Research Institute. The immune induction test group includes a total of 4 groups: buffer, ChimAd used as a mock-up, ChimAd-CV, and ChimAd-CVN. Mice were inoculated intramuscularly twice at 4-week intervals, and blood was collected 4 and 6 weeks after the first priming inoculation.

### B.ELISA; Measurement of antibody titers of mouse anti-serum by ChimAd-CV, ChimAd-CVN

After the mouse immune induction test, it was performed to measure the antibody titer in serum. In a 96-well plate, 100 ng/well of SARS-coronavirus-2 S protein and delta variant S protein were dispensed at a concentration of 2 g/ml and coated at 4 for more than 16 hours. After the reaction, 100 l of PBS containing 1% BSA was dispensed and allowed to react. Each serum was diluted 1:100, then serially diluted 5-fold and reacted at 4 for more than 16 hours. HRP-conjugated goat anti-mouse IgG HRP (Southern Biotech) was diluted 1:3000 and reacted at 37 for 1 hour. After the reaction was completed, the TMB solution (Millipore) was dispensed to proceed with the color development. Afterwards, the reaction was stopped by adding an equal amount of 0.5 N HCl solution (Merck), and the absorbance was measured at a wavelength of 450 nm.

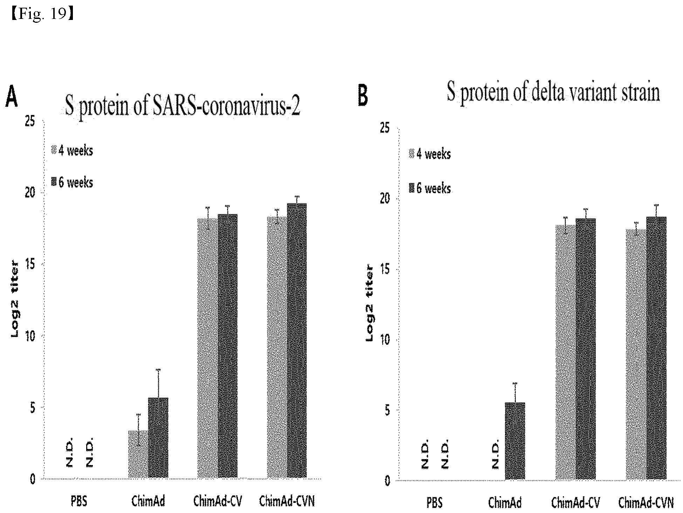

It was confirmed that both the experimental group inoculated with ChimAd-CV and the experimental group inoculated with ChimAd-CVN showed high binding antibody titers of 10⁵ or more against SARS-coronavirus-2 and delta variant strains compared to the control group (FIG. 19).

### C. FRNT (Focus Reduction Neutralization Test); Measurement of neutralizing antibody titers against coronavirus in ChimAd-CV and ChimAd-CVN mouse anti-serum

After the mouse immune induction test, a test was performed to measure neutralizing antibodies in the serum. For the neutralizing antibody test, each mouse serum was first inactivated at 56°Cfor 30 minutes and serially diluted 2-fold using DMEM medium containing 2% FBS. Diluted serum was mixed with 4.5×10² PFU/25µl of SARS-coronavirus-2 coronavirus (NCCP #43326 (BetaCoV/Korea/KCDC03/2020) and 4.0×10²PFU/25µl of delta variant coronavirus (NCCP #43390 (hCoV-19/Korea119861) /KDCA/2021) and reacted for 30 minutes at 37°C After reaction, 50µl of virus and serum mixture was treated with Vero cells (1.5×10⁴ cells/well) cultured one day before and incubated at 37°Cin a CO₂ incubator for 4 hours. After 4 hours of reaction, the supernatant was removed, washed with a PBS buffer solution, and 300 µl of 4% formaldehyde solution (Sigma) was added and fixed in a place without light for more than 16 hours. After the reaction, it was washed with PBS. After washing, 100% cold methanol was added and reacted for 10 minutes, followed by reaction at room temperature for 1 hour using blocking solution. Afterwards, it was treated with anti-SARS-CoV-2 NP rabbit monoclonal antibody (Sino Biological) diluted 1:3000 and incubated at 37°Cfor 1 hour. After incubation, it was washed with a PBS solution containing 0.1% Tween20 (PBS-T), and it was treated with HRP-conjugated goat anti-rabbit IgG (Bio-Rad) diluted 1:2000 and incubated at 37°Cfor 1 hour. After washing with PBS-T, the TMB solution was reacted at room temperature for 30 minutes in the absence of light, the reaction was stopped with water, and then dried. For FRNT50 analysis, a Spot reader (Cytation 7 Cell Imaging Multi-Mode Reader) was used, and the value was calculated according to the dilution factor of the serum. The FRNT method is a method of measuring neutralizing antibodies by identifying virus-infected cells by attaching a virus-derived monoclonal antibody to a virus-infected cell and then attaching a secondary antibody labeled with HRP to develop a color as a substrate.

As a result of the neutralizing antibody against the SARS-coronavirus-2 virus, the neutralizing antibody titer can be confirmed as shown in Table, which is more than 100 times higher than the negative control group in both the ChimAd-CV and ChimAd-CVN groups after the first vaccination. After the second vaccination, neutralizing antibody titers were confirmed to be more than 30 times higher in the ChimAd-CV vaccinated group and more than 50 times higher in the ChimAd-CVN vaccinated group.

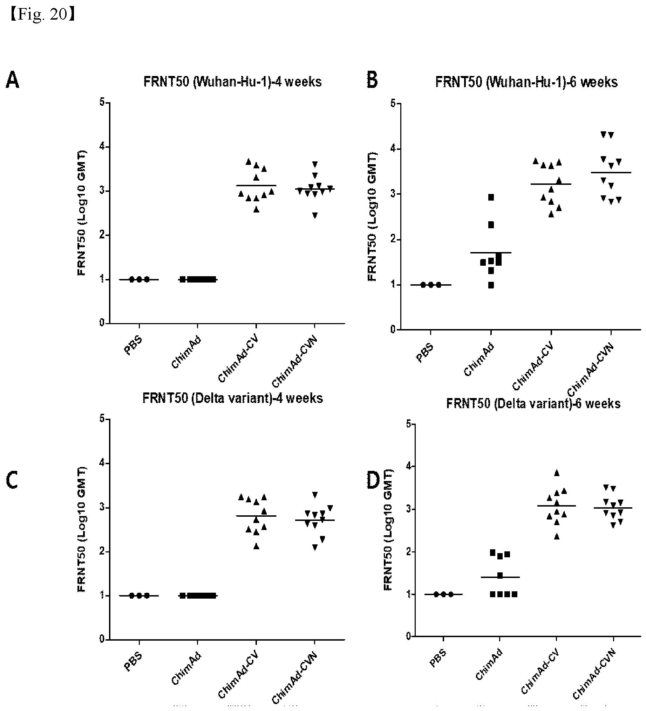

In addition, as a result of neutralizing antibodies against the delta variant virus, it was confirmed that both the ChimAd-CV vaccinated group and the ChimAd-CVN vaccinated group showed neutralizing antibody titers more than 40 times higher than the negative control group after the second vaccination (FIG. 20).

**[TABLE 12]**

| No | Group | SARS-coronavirus-2(FRNT50) | | Delta variant virus (FRNT50) | |
|---|---|---|---|---|---|
| | | 4wk | 6wk | 4wk | 6wk |
| 1 | PBS | 10 | 10 | 10 | 10 |
| 2 | ChimAd | 10 | 50 | 10 | 26 |
| 3 | ChimAd-CV | 1288 | 1660 | 646 | 1202 |
| 4 | ChimAd-CVN | 1096 | 3020 | 513 | 1096 |

### D. Virus challenge test after ChimAd-CV, ChimAd-CVN immunization

The challenge test was intranasal infection with 5×10⁵ PFU SARS-CoV-2 virus (SARS-coronavirus-2, delta variant strain) 4 weeks after boosting vaccination. After vaccination, survival and body weight were monitored daily, and on the 2nd, 4th, and 7th days after infection, blood, lungs, liver, kidneys, and spleen were collected to measure organ weights, and virus titers in the lungs were measured.

As a result of confirming the vaccine protective ability of ChimAd-CV and ChimAd-CVN against SARS-Coronavirus-2 (Wuhan-Hu-1) attack, both survival rates were found to be 100%, and it was confirmed that the ChimAd-CVN vaccinated group did not lose weight and the body weight of the ChimAd-CV vaccinated group was maintained without loss in all but one animal, and that there were individuals whose body weight decreased by more than 10% and then recovered.

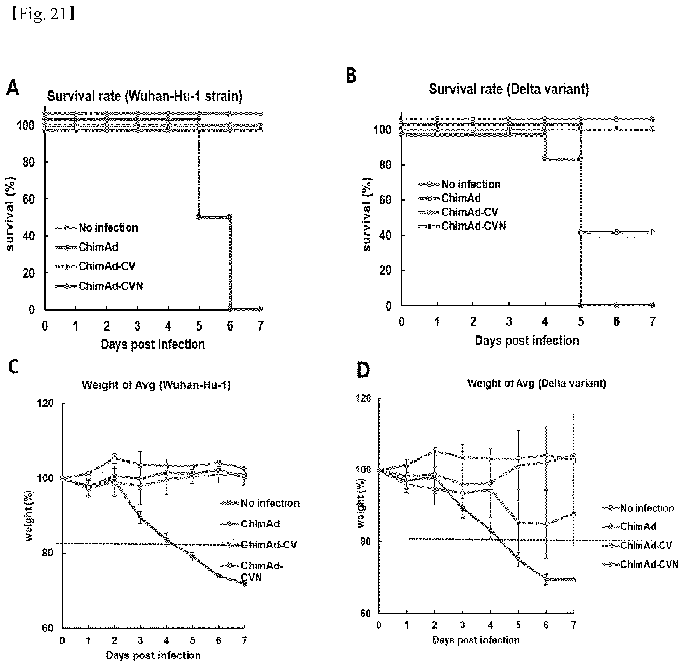

In addition, as a result of confirming the vaccine protective ability of ChimAd-CV and ChimAd-CVN against Delta variant attack, ChimAd-CVN showed a survival rate of 40% and average body weight reduction of about 20% after 7 days of infection. Confirmed what was visible. The ChimAd-CV results showed that the survival rate was 100% and the average body weight was maintained at 100% after 7 days of infection (FIG. 21).

### E. Lung virus titer for ChimAd-CV, ChimAd-CVN immune induction and challenge test (Plaque assay)

After the mouse immune induction and challenge test, a plaque assay test was performed to measure the remaining virus titer in the lungs. To measure titer, Vero cells (ATCC, Cat#CCL-81) were used and cultured in an incubator at 37 and 5% CO₂ for more than 16 hours. The virus isolated from the lungs collected from all groups was stepwise diluted 4-fold up to 10⁶, and 200 l of the diluted virus was added to the cultured Vero cells and reacted at room temperature for 30 minutes. After reaction, the virus mixture was removed, covered with DMEM (Overlay media) containing 0.8% Agarose and 2% FBS, reacted at room temperature for 15 minutes, and then cultured in a 37 , 5% CO₂ incubator for 72 hours. After fixation with 10% formalin solution for 1 hour, overlay media was removed, and plaques were stained for 5 minutes using crystal violet (Sigma). To confirm the virus titer, the number of plaques was counted, each dilution factor and the total amount (ml) were calculated to calculate the final titer.

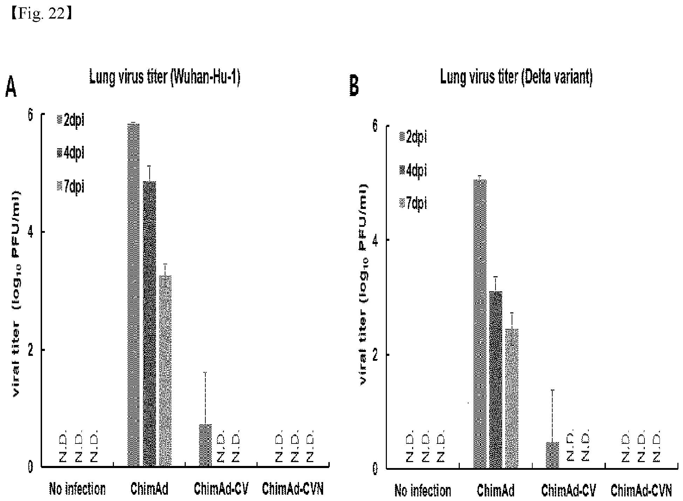

As a result of measuring the residual virus titer in the lungs after the challenge test, in the ChimAd-CV vaccinated group against SARS-coronavirus-2 and delta variant attack, the lung virus titer decreased by more than 20,000 times 2 days after the attack compared to the control group. It was confirmed that the virus was cleared and not measured 4 and 7 days after the attack. It was confirmed that the ChimAd-CVN vaccinated group achieved virus clearance and that the lung virus titer was not measured after 2 days, 4 days, and 7 days (Figure 22).

### Example 9: Application of chimeric adenovirus-based HPV treatment and prevention vaccine

### 9-1) Genetic modification encoding the proteins of HPV16 type E6 and E7 and HPV type 18 E6 and E7 proteins.

For genetic modification of the anti-carcinogenic protein of HPV type 16, in the case of the E6 protein, a deletion mutation of residues 113 to 122 was induced to inhibit the interaction with carcinogenic proteins caused by the binding domain of the anti-cancer protein p53. In the case of the E7 protein, a deletion mutation of residues 21 to 26 was induced to strongly inhibit the interaction with pRb, the main target, and to suppress the transforming activity ability of E7 itself. In addition, for genetic modification of the anti-cancer protein of HPV type 18, in the case of the E6 protein, a deletion mutation of residues 113 to 122 was induced to inhibit the interaction with carcinogenic proteins caused by the binding domain of the anti-cancer protein p53. In the case of the E7 protein, a deletion mutation of residues 24 to 29 was induced to strongly inhibit the interaction with pRb, the main target, and to suppress the transforming activity ability of E7 itself.

Linear polypeptide (E6E7) in which the amino acids of E6 and E7, tumor proteins derived from human papillomavirus type 16, have been deleted, and linear polypeptide (Linear; E6E7) in which the amino acids of E6 and E7, tumor proteins derived from human papillomavirus type 18, have been deleted, were combined with Linker.

### 9-2) Chimeric adenovirus-based HPV type 16, type 18 E6, E7 gene synthesis and chimeric adenovirus cloning

The E6 and E7 genes, which are anticancer proteins of human papillomavirus types 16 and 18, were modified and synthetic genes encoding these proteins were obtained. For each protein, the strain was selected as shown in Table 13 and the codon was optimized to facilitate expression in HEK cells.

**[TABLE 13]**

| | **Accession no.** |
|---|---|
| HPV 16E6E7 | E6; AAV91660.1, E7; BAO18680.1 |
| HPV 18 E6E7 | E6; 1ADC35716.1, E7; 1ADC35717.1 |

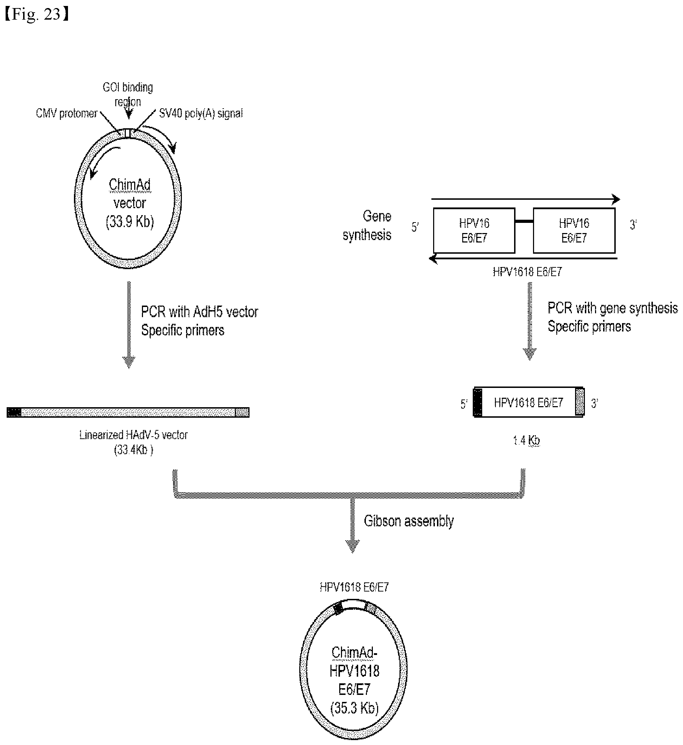

Cloning of the HPV vaccine antigen cloned into the chimeric adenovirus vector was performed in the same manner as the SARS-coronavirus vaccine production (Example 8-3), and finally the ChimAd-HPV plasmid was obtained (Figure 23).

**[TABLE 14]**

| **Primer name** | **Sequence (5' to 3' direction)** | **PCR product** |
|---|---|---|
| ChimA d vector | F: CTAAGCTTCTAGATAAGATATCCGATCCAC (SEQ ID NO: 59) | 33.4 Kb |
| | R: CGCGTCGACGGTACCAGATCTCTAGCGGATC (SEQ ID NO: 60) | |
| HPV16/ 18 E6E7 | F: GATCTGGTACCGTCGACGCGATGCACCAGAAGAGAACA (SEQ ID NO: 61) | 1.4Kb |
| | R: TATCTTATCTAGAAGCTTAGTTATTGTTGACTAGCGCACCA TGG (SEQ ID NO: 62) | |

### 9-3) Immunization induction using C57BL/6 female

Mouse immunization was performed using the ChimAd-HPV vaccine candidate obtained by the above preparing method. The test group includes three groups: buffer, ChimAd used as a mock-up, and ChimAd-HPV. For the immune induction test, 5-week-old C57BL/6 female mice were used, and each adenovirus test group was injected at a concentration of 2*10^7 PFU/ml. At 4-week intervals, the first and second vaccinations were administered by intramuscular injection twice (days 0 and 14), and blood was collected and serum was separated and the spleen was removed at 2 and 4 weeks after the first vaccination (days 14 and 28). In addition, to confirm the effect of treating cervical cancer, 1×10⁵ TC-1 tumor cells were subcutaneously injected into the flank of 6-week-old C57BL/6 female mice 7 days before the first vaccination, and 6 mice were used for each group.. In addition, to confirm the effect of preventing cervical cancer, 1×10⁵ TC-1 tumor cells were subcutaneously injected into the flanks of 6-week-old C57BL/6 female mice 7 days after the second vaccination, and 6 mice were used in each group.

### 9-4) ELISPOT; Specific T cell immune response by ChimAd-HPV adenovirus

On the 35th day after the second vaccination, spleens were removed from each group. The spleen was crushed into small pieces using a Cell strainer (Falcon) and the spleen cells were recovered and washed with a PBS buffer solution. Afterwards, red blood cells were removed using red blood cell lysis buffer (Sigma), and the number of spleen cells was counted and diluted to the number of cells to be used in the test. This experiment used R&D system's mouse IFN-gamma ELISpot kit, and the test was performed according to the manufacturer's instructions.

Then, 2×10⁵ cells and 4×10⁴ cells of splenocytes recovered from each mouse and the 49-57 amino acid peptide of Genscript's HPV E7 protein were used as stimulating antigens on a plate coated with mouse IFN-gamma monoclonal antibody and incubated for 16 hours at 37°Cin a 5% CO₂ incubator. After incubation, the cells were washed four times using 1X Wash buffer, 100µl of streptavidin-AKP (alkaline phosphate) was added and incubated at room temperature for 2 hours. After the final washing, 100 µl of BCIP/NBT substrate was added and incubated at room temperature for 45 minutes. When color development due to the reaction appeared, it was washed with sterilized water and the number of colored dots was counted.

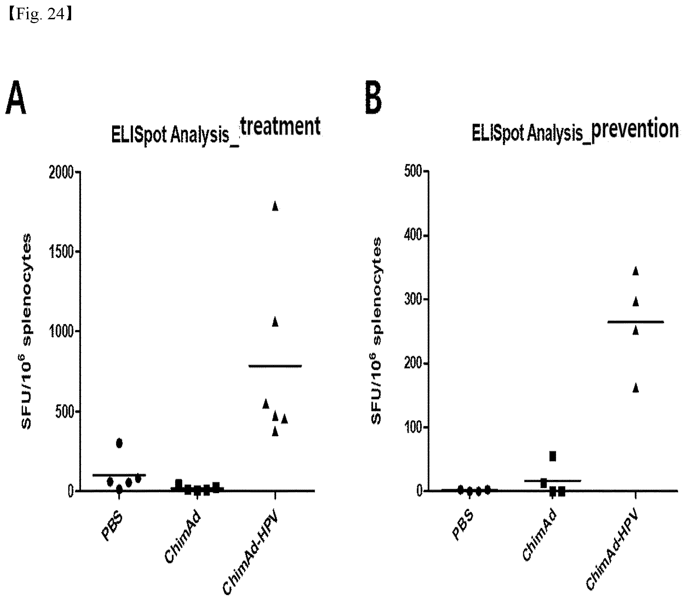

As a result of measuring the specific T cell immune response to the HPV E7 protein antigen using ELISPOT, the ChimAd-HPV group showed higher results than the control group, confirming that it induced a specific immune response to the antigen. (Figure 24)

### 9-5) Tumor inhibition assay; Confirmation of tumor treatment and prevention effects by ChimAd-HPV adenovirus

To confirm the treatment effect of ChimAd-HPV adenovirus on cervical cancer, 1×10⁵ TC-1 tumor cells were subcutaneously injected into the flank of 6-week-old C57BL/6 female mice, and 6 mice were used per group. To confirm treatment efficacy, 7 days after injection of TC-1 cells, intramuscular injection of control group and ChimAd-HPV adenovirus was performed, and a second injection was performed on the 21 st day after injection of tumor cells. In addition, to confirm the preventive efficacy, control and ChimAd-HPV adenoviruses were injected intramuscularly twice at two-week intervals, and 14 days after the second injection, TC-1 tumor cells (1×10⁵ cells) were injected subcutaneously into the flank. Tumor cells were measured every 4-5 days by measuring their longest (length) and shortest dimensions (width) using digital calipers to estimate tumor size in mice. Tumor volume was calculated by the following equation: tumor volume = (length × width²)/2.

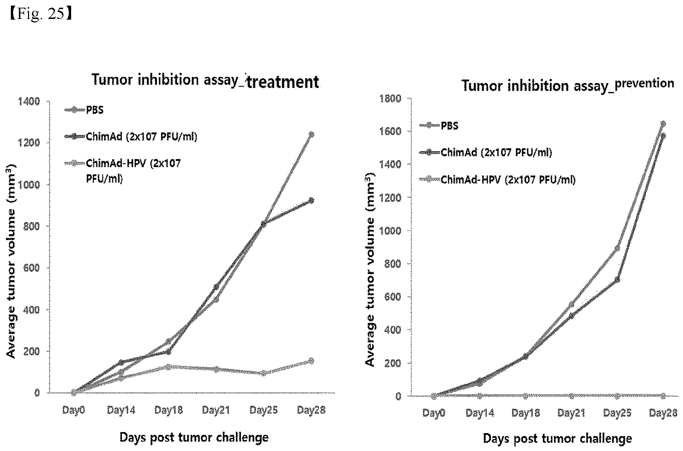

As shown in Figure 24, as a result of measuring the tumor volume using ChimAd-HPV adenovirus, the tumor volume was found to be lower in the ChimAd-HPV adenovirus group compared to the negative control group and positive control group in both therapeutic and preventive efficacy. It was confirmed that no tumor cells occurred in the ChimAd-HPV adenovirus group. (Figure 25)

### [Sequence list pre text]

SEQ ID NO: 1- Chimpanzee adenovirus serotype 6 Fiber knob domain a.a sequence(synthesized ChAdV-6 knob a. a sequences are underlined) 
PDPSPNCQLLSDRDAKFTLCLTKCGSQILGTVAVAAVTVGSALNPINDTVKSAIVFLR

FDSDGVLMSNSSMVGDYWNFREGQTTQSVAYTNAVGFMPNIGAYPKTQSKTPKNSI

VSQVYLTGETTMPMTLTITFNGTDEKDTTPVSTYSMTFTWQWTGDYKDKNITFATN

SFS
SEQ ID NO: 2- Chimpanzee adenovirus serotype 6/Pan6/Sad23 Fiber knob domain nucleotide sequence 
CCTGACCCAAGCCCTAATTGTCAATTACTTTCAGACAGAGATGCCAAATTTACTC

TCTGTCTTACAAAATGCGGTAGTCAAATACTAGGCACTGTGGCAGTGGCGGCTGT

TACTGTAGGATCAGCACTAAATCCAATTAATGACACAGTCAAAAGCGCCATAGT

TTTCCTTAGATTTGATTCCGATGGTGTACTCATGTCAAACTCATCAATGGTAGGTG

ATTACTGGAACTTTAGGGAGGGACAGACCACTCAAAGTGTAGCCTATACAAATG

CTGTGGGATTCATGCCAAATATAGGTGCATATCCAAAAACCCAAAGTAAAACAC

CTAAAAATAGCATAGTCAGTCAGGTATATTTAACTGGAGAAACTACTATGCCAAT

GACACTAACCATAACTTTCAATGGCACTGATGAAAAAGACACAACCCCAGTTAG
 
CACCTACTCTATGACTTTTACATGGCAGTGGACTGGAGACTATAAGGACAAAAAT

ATTACCTTTGCTACCAACTCATTCTCT
SEQ ID NO: 3- Chimeric hexon HVR 1 to 7 region a.a sequence (HAdV-5 hexon + HAdV-28 HVR 1-7 region) 
SSQWDAQEKSGQGSDMVTKTHTFGVAAMGGENITKNGLQIGTEITADNQKKEIFAN

KTYQPEPQVGEENWQENEVFYGGRALKKETKMKPCYGSFARPTNENGGQAKFKTP

AEGQEPKELDIDLAFFDTDGGTADTEYKADIVMYAENVNLETPDTHVVYKPGKEDD

SSEINLVQQSMPNRPNYIGFRDNFVGLMYYNSTGNMGVLAGQASQLNAVVDLQDR

NTELSYQLLLDSLGDRTRYFSMWNSAVDSYDPDVRIIENHGVEDELPNYCFPLDGLG

TNATYQGVKVSTGDGATQSGWAKDDTMARQNQICRGNIY
SEQ ID NO: 4- Chimeric hexon HVR 1 to 7 region nucleotide sequence (HAdV-5 hexon + HAdV-28 HVR 1-7 region) 
TCCAGTCAGTGGGATGCGCAAGAAAAAAGTGGACAAGGAAGTGACATGG

TTACTAAAACTCACACATTTGGGCAGGCGCCTTATTCTGGTATAAATATTACAAA

GGAGGGTATTCAAATAGGTACTGAAATTACCGCCGACAATCAAAAGAAAGAAAT

TTTTGCCGATAAAACATTTCAACCTGAACCTCAAATAGGAGAAGAGAACTGGCA

AGAAAATGAAGTCTTCTATGGCGGGAGAGTCCTAAAAAAGACTACCCCAATGAA
 
ACCATGTTACGGTTCATATGCAAAACCCACAAATGAAAATGGAGGGCAAGCAAA

ATTCAAAACACCTGCTGAAGGACAGGAACCTAAAGAACTTGACATTGACCTTGC

TTTTTTCGACACTGACGGCGGAACAGCTGACACTGAATATAAAGCAGATATTGTA

TTGTACAGTGAAGATGTAGATATAGAAACCCCAGACACTCATATTTCTTACATGC

CCGGCAAGGAAGATGACAGTTCAGAAATCAATCTAGTTCAACAATCTATGCCCA

ACAGGCCTAATTACATTGCTTTTAGGGACAATTTTATTGGTCTAATGTATTACAAC

AGCACGGGTAATATGGGTGTTCTGGCGGGCCAAGCATCGCAGTTGAATGCTGTT

GTAGATTTGCAAGACAGAAACACAGAGCTTTCATACCAGCTTTTGCTTGATTCCA

TTGGTGATAGAACCAGGTACTTTTCTATGTGGAATCAGGCTGTTGACAGCTATGA

TCCAGATGTTAGAATTATTGAAAATCATGGAACTGAAGATGAACTTCCAAATTAC

TGCTTTCCACTGGACGGATTGGGAACTAATGCTACCTATCAAGGTGTAAAAGTAT

CAACTGGAGATGGTGCTACGCAAAGCGGATGGGCAAAAGACGACACAATGGCC

AGGCAAAACCAAATATGCAGGGGTAACATCTAC
SEQ ID NO: 5- Chimeric fiber gene sequence (HAdV-5 tail, shift domain + ChAdV-6 knob domain) 
ATGAAGCGCGCAAGACCGTCTGAAGATACCTTCAACCCCGTGTATCCATATGAC
ACGGAAACCGGTCCTCCAACTGTGCCTTTTCTTACTCCTCCCTTTGTATCCCCCAA
TGGGTTTCAAGAGAGTCCCCCTGGGGTACTCTCTTTGCGCCTATCCGAACCTCTA
GTTACCTCCAATGGCATGCTTGCGCTCAAAATGGGCAACGGCCTCTCTCTGGACG
AGGCCGGCAACCTTACCTCCCAAAATGTAACCACTGTGAGCCCACCTCTCAAAA
 
AAACCAAGTCAAACATAAACCTGGAAATATCTGCACCCCTCACAGTTACCTCAG
AAGCCCTAACTGTGGCTGCCGCACCTCTAATGGTCGCGGGCAACACACTCAC
CATGCAATCACAGGCCCCGCTAACCGTGCACGACTCCAAACTTAGCATTGCCACC
CAAGGACCCCTCACAGTGTCAGAAGGAAAGCTAGCCCTGCAAACATCAGGCCCC
CTCACCACCACCGATAGCAGTACCCTTACTATCACTGCCTCACCCCCTCTAACTA
CTGCCACTGGTAGCTTGGGCATTGACTTGAAAGAGCCCATTTATACACAAAATGG
AAAACTAGGACTAAAGTACGGGGCTCCTTTGCATGTAACAGACGACCTAAACAC
TTTGACCGTAGCAACTGGTCCAGGTGTGACTATTAATAATACTTCCTTGCAAACT
AAAGTTACTGGAGCCTTGGGTTTTGATTCACAAGGCAATATGCAACTTAATGTAG
CAGGAGGACTAAGGATTGATTCTCAAAACAGACGCCTTATACTTGATGTTAGTTA
TCCGTTTGATGCTCAAAACCAACTAAATCTAAGACTAGGACAGGGCCCTCTTTTT
ATAAACTCAGCCCACAACTTGGATATTAACTACAACAAAGGCCTTTACTTGTTTA
CAGCTTCAAACAATTCCAAAAAGCTTGAGGTTAACCTAAGCACTGCCAAGGGGT
TGATGTTTGACGCTACAGCCATAGCCATTAATGCAGGAGATGGGCTTGAATTTGG
TTCACCTAATGCACCAAACACAAATCCCCTCAAAACAAAAATTGGCCATGGCCT
AGAATTTGATTCAAACAAGGCTATGGTTCCTAAACTAGGAACTGGCCTTAGTTTT
GACAGCACAGGTGCCATTACAGTAGGAAACAAAAATAATGATAAGCTAACTTTG
TGGACCACACCTGACCCAAGCCCTAATTGTCAATTACTTTCAGACAGAGATGCCA
AATTTACTCTCTGTCTTACAAAATGCGGTAGTCAAATACTAGGCACTGTGGCAGT
GGCGGCTGTTACTGTAGGATCAGCACTAAATCCAATTAATGACACAGTCAAAAG
CGCCATAGTTTTCCTTAGATTTGATTCCGATGGTGTACTCATGTCAAACTCATCAA
TGGTAGGTGATTACTGGAACTTTAGGGAGGGACAGACCACTCAAAGTGTAGCCT
ATACAAATGCTGTGGGATTCATGCCAAATATAGGTGCATATCCAAAAACCCAAA
GTAAAACACCTAAAAATAGCATAGTCAGTCAGGTATATTTAACTGGAGAAACTA
CTATGCCAATGACACTAACCATAACTTTCAATGGCACTGATGAAAAAGACACAA
CCCCAGTTAGCACCTACTCTATGACTTTTACATGGCAGTGGACTGGAGACTATAA
GGACAAAAATATTACCTTTGCTACCAACTCATTCTCT
SEQ ID NO: 6- Chimeric hexon gene sequence (HAdV-5 hexon + HAdV-28 HVR 1-7region) 
ATGGCTACCCCTTCGATGATGCCGCAGTGGTCTTACATGCACATCTCGGGCCAGG

ACGCCTCGGAGTACCTGAGCCCCGGGCTGGTGCAGTTTGCCCGCGCCACCGAGA

CGTACTTCAGCCTGAATAACAAGTTTAGAAACCCCACGGTGGCGCCTACGCACG

ACGTGACCACAGACCGGTCCCAGCGTTTGACGCTGCGGTTCATCCCTGTGGACCG

TGAGGATACTGCGTACTCGTACAAGGCGCGGTTCACCCTAGCTGTGGGTGATAAC
 
CGTGTGCTGGACATGGCTTCCACGTACTTTGACATCCGCGGCGTGCTGGACAGGG
GCCCTACTTTTAAGCCCTACTCTGGCACTGCCTACAACGCCCTGGCTCCCAAGGG
TGCCCCAAATTCCAGTCAGTGGGATGCGCAAGAAAAAAGTGGACAAGGAAGTGA
CATGGTTACTAAAACTCACACATTTGGGCAGGCGCCTTATTCTGGTATAAATATT
ACAAAGGAGGGTATTCAAATAGGTACTGAAATTACCGCCGACAATCAAAAGAAA
GAAATTTTTGCCGATAAAACATTTCAACCTGAACCTCAAATAGGAGAAGAGAAC
TGGCAAGAAAATGAAGTCTTCTATGGCGGGAGAGTCCTAAAAAAGACTACCCCA
ATGAAACCATGTTACGGTTCATATGCAAAACCCACAAATGAAAATGGAGGGCAA
GCAAAATTCAAAACACCTGCTGAAGGACAGGAACCTAAAGAACTTGACATTGAC
CTTGCTTTTTTCGACACTGACGGCGGAACAGCTGACACTGAATATAAAGCAGATA
TTGTATTGTACAGTGAAGATGTAGATATAGAAACCCCAGACACTCATATTTCTTA
CATGCCCGGCAAGGAAGATGACAGTTCAGAAATCAATCTAGTTCAACAATCTAT
GCCCAACAGGCCTAATTACATTGCTTTTAGGGACAATTTTATTGGTCTAATGTATT
ACAACAGCACGGGTAATATGGGTGTTCTGGCGGGCCAAGCATCGCAGTTGAATG
CTGTTGTAGATTTGCAAGACAGAAACACAGAGCTTTCATACCAGCTTTTGCTTGA
TTCCATTGGTGATAGAACCAGGTACTTTTCTATGTGGAATCAGGCTGTTGACAGC
TATGATCCAGATGTTAGAATTATTGAAAATCATGGAACTGAAGATGAACTTCCAA
 
ATTACTGCTTTCCACTGGACGGATTGGGAACTAATGCTACCTATCAAGGTGTAAA
AGTATCAACTGGAGATGGTGCTACGCAAAGCGGATGGGCAAAAGACGACACAAT
GGCCAGGCAAAACCAAATATGCAGGGGTAACATCTACGCCATGGAAATCAATCT
AAATGCCAACCTGTGGAGAAATTTCCTGTACTCCAACATAGCGCTGTATTTGCCC
GACAAGCTAAAGTACAGTCCTTCCAACGTAAAAATTTCTGATAACCCAAACACCT
ACGACTACATGAACAAGCGAGTGGTGGCTCCCGGGCTAGTGGACTGCTACATTA
ACCTTGGAGCACGCTGGTCCCTTGACTATATGGACAACGTCAACCCATTTAACCA
CCACCGCAATGCTGGCCTGCGCTACCGCTCAATGTTGCTGGGCAATGGTCGCTAT
GTGCCCTTCCACATCCAGGTGCCTCAGAAGTTCTTTGCCATTAAAAACCTCCTTCT
CCTGCCGGGCTCATACACCTACGAGTGGAACTTCAGGAAGGATGTTAACATGGTT
CTGCAGAGCTCCCTAGGAAATGACCTAAGGGTTGACGGAGCCAGCATTAAGTTT
GATAGCATTTGCCTTTACGCCACCTTCTTCCCCATGGCCCACAACACCGCCTCCA
CGCTTGAGGCCATGCTTAGAAACGACACCAACGACCAGTCCTTTAACGACTATCT
CTCCGCCGCCAACATGCTCTACCCTATACCCGCCAACGCTACCAACGTGCCCATA
TCCATCCCCTCCCGCAACTGGGCGGCTTTCCGCGGCTGGGCCTTCACGCGCCTTA
AGACTAAGGAAACCCCATCACTGGGCTCGGGCTACGACCCTTATTACACCTACTC
TGGCTCTATACCCTACCTAGATGGAACCTTTTACCTCAACCACACCTTTAAGAAG
 
GTGGCCATTACCTTTGACTCTTCTGTCAGCTGGCCTGGCAATGACCGCCTGCTTAC
CCCCAACGAGTTTGAAATTAAGCGCTCAGTTGACGGGGAGGGTTACAACGTTGC
CCAGTGTAACATGACCAAAGACTGGTTCCTGGTACAAATGCTAGCTAACTATAAC
ATTGGCTACCAGGGCTTCTATATCCCAGAGAGCTACAAGGACCGCATGTACTCCT
TCTTTAGAAACTTCCAGCCCATGAGCCGTCAGGTGGTGGATGATACTAAATACAA
GGACTACCAACAGGTGGGCATCCTACACCAACACAACAACTCTGGATTTGTTGG
CTACCTTGCCCCCACCATGCGCGAAGGACAGGCCTACCCTGCTAACTTCCCCTAT
CCGCTTATAGGCAAGACCGCAGTTGACAGCATTACCCAGAAAAAGTTTCTTTGCG
ATCGCACCCTTTGGCGCATCCCATTCTCCAGTAACTTTATGTCCATGGGCGCACTC
ACAGACCTGGGCCAAAACCTTCTCTACGCCAACTCCGCCCACGCGCTAGACATG
ACTTTTGAGGTGGATCCCATGGACGAGCCCACCCTTCTTTATGTTTTGTTTGAAGT
CTTTGACGTGGTCCGTGTGCACCAGCCGCACCGCGGCGTCATCGAAACCGTGTAC
CTGCGCACGCCCTTCTCGGCCGGCAACGCCACAACATAA
 [ChimAd-based coronavirus vaccine sequence]
SEQ ID NO: 7 - SARS-CoV-2_B.1.351(beta) spike modified amino acid sequence 
MFVFLVLLPLVSSQCVNFTTRTQLPPAYTNSFTRGVYYPDKVFRSSVLHSTQDLFLPF

FSNVTWFHAIHVSGTNGTKRFANPVLPFNDGVYFASTEKSNIIRGWIFGTTLDSKTQS
 
LLIVNNATNVVIKVCEFQFCNDPFLGVYYHKNNKSWMESEFRVYSSANNCTFEYVS

QPFLMDLEGKQGNFKNLREFVFKNIDGYFKIYSKHTPINLVRGLPQGFSALEPLVDLP

IGINITRFQTLHISYLTPGDSSSGWTAGAAAYYVGYLQPRTFLLKYNENGTITDAVDC

ALDPLSETKCTLKSFTVEKGIYQTSNFRVQPTESIVRFPNITNLCPFGEVFNATRFASV

YAWNRKRISNCVADYSVLYNSASFSTFKCYGVSPTKLNDLCFTNVYADSFVIRGDEV

RQIAPGQTGNIADYNYKLPDDFTGCVIAWNSNNLDSKVGGNYNYLYRLFRKSNLKP

FERDISTEIYQAGSTPCNGVKGFNCYFPLQSYGFQPTYGVGYQPYRVVVLSFELLHAP

ATVCGPKKSTNLVKNKCVNFNFNGLTGTGVLTESNKKFLPFQQFGRDIADTTDAVR

DPQTLEILDITPCSFGGVSVITPGTNTSNQVAVLYQGVNCTEVPVAIHADQLTPTWRV

YSTGSNVFQTRAGCLIGAEHVNNSYECDIPIGAGICASYQTQTNSPQQAQSVASQSIIA

YTMSLGVENSVAYSNNSIAIPTNFTISVTTEILPVSMTKTSVDCTMYICGDSTECSNLL

LQYGSFCTQLNRALTGIAVEQDKNTQEVFAQVKQIYKTPPIKDFGGFNFSQILPDPSK

PSKRSFIEDLLFNKVTLADAGFIKQYGDCLGDIAARDLICAQKFNGLTVLPPLLTDEMI

AQYTSALLAGTITSGWTFGAGAALQIPFAMQMAYRFNGIGVTQNVLYENQKLIANQ

FNSAIGKIQDSLSSTASALGKLQDVVNQNAQALNTLVKQLSSNFGAISSVLNDILSRL

DPPEAEVQIDRLITGRLQSLQTYVTQQLIRAAEIRASANLAATKMSECVLGQSKRVDF

CGKGYHLMSFPQSAPHGVVFLHVTYVPAQEKNFTTAPAICHDGKAHFPREGVFVSN
 
GTHWFVTQRNFYEPQIITTDNTFVSGNCDVVIGIVNNTVYDPLQPELDSFKEELDKYF

KNHTSPDVDLGDISGINASVVNIQKEIDRLNEVAKNLNESLIDLQELGKYEQYIKWPW

YIWLGFIAGLIAIVMVTIMLCCMTSCCSCLKGCCSCGSCCKFDEDDSEPVLKGVKLH

YTSEQ
SEQ ID NO: 8 - SARS-CoV-2_B.1.351(beta) spike modified nucleic acid sequence

ATG TTT GTG TTT CTG GTG CTG CTG CCT CTG GTG AGC AGC CAG TGC GTG A

AC TTC ACC ACC AGA ACC CAG CTG CCT CCT GCC TAC ACC AAC AGC TTT AC

C AGA GGC GTG TAT TAC CCC GAC AAA GTG TTT AGA TCC TCC GTG CTG CAC

AGC ACC CAG GAC CTG TTT CTG CCC TTT TTC AGC AAC GTG ACC TGG TTC C

AT GCC ATC CAC GTG AGC GGC ACC AAC GGC ACC AAG AGA TTC GCC AAT C

CC GTG CTG CCT TTT AAT GAC GGC GTG TAC TTT GCC AGC ACC GAG AAG AG

C AAT ATC ATC AGA GGC TGG ATC TTC GGC ACC ACC CTG GAT AGC AAG ACC

CAG AGC CTG CTG ATC GTG AAT AAT GCC ACC AAT GTG GTG ATC AAA GTG

TGC GAG TTC CAG TTT TGT AAC GAC CCC TTT CTG GGC GTG TAT TAC CAC A

AG AAC AAC AAA AGC TGG ATG GAG AGC GAA TTC AGA GTG TAC AGC AGC G

CC AAT AAC TGC ACC TTT GAG TAC GTG AGC CAG CCC TTT CTG ATG GAC CT

G GAG GGC AAG CAG GGC AAC TTC AAG AAC CTG AGA GAG TTC GTG TTT AA

A AAT ATC GAC GGC TAC TTC AAG ATC TAC AGC AAG CAT ACA CCC ATC AAC
CTG GTG AGA GGC CTG CCC CAG GGC TTC AGC GCC CTG GAG CCC CTG GTG
GAC CTG CCC ATC GGC ATC AAC ATT ACC CGG TTT CAG ACC CTG CAC AGA A
GC TAT CTG ACC CCC GGC GAC AGC AGC AGC GGC TGG ACC GCC GGA GCC G
CC GCC TAT TAT GTG GGC TAC CTG CAG CCC AGA ACC TTC CTG CTG AAA TA
C AAC GAG AAC GGC ACC ATC ACC GAC GCC GTG GAC TGC GCC CTG GAC CC
C CTG AGC GAG ACC AAG TGT ACC CTG AAG AGC TTT ACC GTG GAG AAG GG
C ATC TAC CAG ACC AGC AAC TTT AGA GTG CAG CCC ACC GAG AGC ATC GT
G AGA TTT CCC AAC ATC ACC AAC CTG TGC CCC TTC GGG GAG GTG TTT AAT
GCC ACC AGA TTC GCC AGC GTG TAC GCC TGG AAT AGA AAA AGA ATC AGC
AAC TGC GTG GCC GAC TAC AGC GTG CTG TAC AAC TCT GCC AGC TTT TCC A
CC TTT AAG TGC TAC GGC GTG AGC CCA ACC AAA CTG AAC GAC CTG TGC TT
C ACC AAC GTG TAC GCC GAC AGC TTC GTG ATC AGA GGC GAC GAG GTG AG
A CAG ATC GCC CCC GGC CAG ACC GGC AAT ATC GCC GAT TAC AAC TAC AA
G CTG CCC GAC GAC TTT ACC GGC TGC GTG ATC GCC TGG AAC AGC AAC AAC
CTG GAC AGC AAG GTG GGC GGC AAC TAC AAC TAC CTG TAC AGA CTG TTT
AGA AAG AGC AAC CTG AAG CCC TTT GAG AGA GAC ATC AGC ACC GAG ATC

TAC CAG GCC GGC AGC ACC CCC TGC AAC GGC GTG AAA GGC TTT AAT TGT T

AC TTC CCA CTG CAG TCC TAC GGC TTC CAG CCT ACC TAC GGC GTG GGA TA

T CAG CCC TAT AGA GTG GTG GTG CTG TCC TTC GAG CTG CTG CAC GCC CCC

GCC ACC GTG TGC GGC CCA AAG AAG AGC ACC AAC CTG GTG AAG AAC AAG

TGC GTG AAC TTC AAC TTC AAC GGC CTG ACC GGC ACC GGC GTG CTG ACC G

AG TCC AAC AAG AAA TTT CTG CCC TTC CAG CAG TTC GGA AGA GAC ATT GC

C GAT ACC ACC GAC GCC GTG AGA GAC CCC CAG ACC CTG GAG ATC CTG GA

C ATC ACC CCC TGT AGC TTC GGC GGC GTG TCC GTG ATC ACC CCA GGC ACC

AAC ACC TCC AAC CAG GTG GCC GTG CTG TAC CAG GGC GTG AAC TGT ACC

GAG GTG CCT GTG GCC ATC CAC GCC GAT CAG CTG ACC CCA ACC TGG AGA

GTG TAC TCC ACC GGC TCC AAC GTG TTC CAG ACC AGA GCC GGC TGT CTG A

TC GGC GCC GAG CAC GTG AAC AAC TCC TAC GAG TGC GAC ATC CCT ATC GG

C GCC GGC ATC TGC GCC AGC TAC CAG ACC CAG ACC AAT AGC CCC CAG

CAG GCC CAG

TCC GTG GCC AGC CAG TCC ATC ATT GCC TAC ACC ATG TCC CTG GGC GTG G

AG AAC AGC GTG GCC TAC TCC AAC AAC AGC ATC GCC ATC CCC ACC AAT TT

C ACC ATC AGC GTG ACC ACC GAA ATC CTG CCA GTG AGC ATG ACC AAA AC

C TCC GTG GAT TGC ACC ATG TAC ATC TGC GGA GAC TCC ACC GAG TGC TCC

AAC CTG CTG CTG CAG TAC GGC TCC TTT TGC ACC CAG CTG AAC AGA GCC C

TG ACC GGA ATC GCC GTG GAG CAG GAT AAA AAC ACC CAG GAA GTG TTC G

CC CAG GTG AAA CAG ATC TAC AAG ACC CCC CCC ATC AAA GAT TTT GGA G

GC TTT AAT TTT AGC CAG ATC CTG CCT GAT CCC TCC AAA CCC AGC AAG AG

A TCC TTT ATC GAG GAT CTG CTG TTC AAC AAA GTG ACA CTG GCC GAT GCC

GGA TTT ATC AAA CAG TAC GGA GAC TGC CTG GGC GAC ATC GCC GCC AGA

GAC CTG ATC TGC GCC CAG AAG TTT AAC GGC CTG ACC GTG CTG CCA CCC C

TG CTG ACC GAC GAG ATG ATC GCC CAG TAT ACC TCC GCC CTG CTG GCC GG

C ACC ATC ACC TCC GGC TGG ACC TTT GGC GCC GGA GCC GCC CTG CAG ATC

CCA TTT GCC ATG CAG ATG GCC TAC AGA TTT AAT GGA ATC GGA GTG ACC C

AG AAC GTG CTG TAT GAG AAC CAG AAA CTG ATC GCC AAT CAG TTC AAC A

GC GCC ATC GGC AAG ATC CAG GAT TCT CTG AGC TCC ACC GCC TCT GCC CT

G GGC AAG CTG CAG GAC GTG GTG AAT CAG AAC GCC CAG GCC CTG AAC AC

C CTG GTG AAA CAG CTG AGC AGC AAC TTT GGC GCC ATC AGC AGC GTG CT

G AAT GAT ATC CTG AGC AGA CTG GAT CCT CCA GAG GCC GAG GTG CAG AT

C GAC AGA CTG ATC ACA GGG AGA CTG CAG AGC CTG CAG ACC TAT GTG AC

A CAG CAG CTG ATC AGA GCC GCC GAA ATT AGA GCC AGC GCC AAC CTG GC

C GCC ACC AAA ATG AGC GAG TGT GTG CTG GGA CAG AGC AAA AGA GTG GA

C TTC TGC GGC AAA GGC TAC CAT CTG ATG AGC TTC CCC CAG AGC GCC CCC

CAC GGC GTG GTG TTC CTG CAT GTG ACC TAC GTG CCC GCC CAG GAA AAA A

AT TTT ACC ACC GCC CCT GCC ATC TGT CAC GAC GGC AAA GCC CAT TTT CC

C AGA GAG GGC GTG TTT GTG AGC AAC GGC ACC CAC TGG TTT GTG ACC CA

G AGA AAC TTT TAC GAA CCC CAG ATC ATC ACC ACA GAT AAT ACC TTT GTG

AGC GGA AAT TGT GAT GTG GTG ATC GGC ATC GTG AAT AAT ACC GTG TAT G

AT CCT CTG CAG CCA GAG CTG GAC AGC TTT AAG GAG GAG CTG GAC AAG T

AT TTT AAA AAT CAT ACC AGC CCC GAC GTG GAT CTG GGA GAT ATC AGC GG

A ATC AAC GCC TCC GTG GTG AAT ATC CAG AAG GAG ATC GAC AGA CTG AA

C GAG GTG GCC AAA AAT CTG AAT GAG TCC CTG ATC GAC CTG CAG GAG CT

G GGC AAA TAT GAA CAG TAC ATC AAG TGG CCA TGG TAC ATC TGG CTG GG

G TTT ATC GCC GGA CTG ATC GCC ATC GTG ATG GTG ACC ATC ATG CTG TGT

TGC ATG ACC AGC TGT TGT AGC TGT CTG AAA GGC TGT TGT AGC TGC GGC A

GC TGC TGC AAA TTT GAC GAG GAT GAT TCC GAG CCA GTG CTG AAG GGA G

TG AAA CTG CAC TAT ACC TGA

The following describes the sequence of the fusion CVN polypeptide insert of the invention.

MGLPNNTASWFTALTQHGKEDLKFPRGQGVPINTNSSPDDQIGYYRRATRRIRGGDG

KMKDLSPRWYFYYLGTGPEAGLPYGANKDGIIWVATEGALNTPKDHIGTRNPANNA

AIVLQLPQGTTLPKGFYAEGSRGGS (SEQ ID NO: 9)

GGGGSGGGGSGGGGS↓GGGGSGGGGSGGGGSTKKSAAEASKKPRQKRTATKAYNV

TQAFGRRGPEQTQGNFGDQELIRQGTDYKHWPQIAQFAPSASAFFGMSRIGMEVTPS

GTWLTYTGAIKLDDKDPNFKDQVILLNKHIDAYKTFP* (SEQ ID NO: 10)

In the above sequence, SEQ ID NO: 9 is the sequence bound to the N-terminus of the SARS-CoV-2-B.1.1.351 Spike protein, SEQ ID NO: 10 is the sequence bound to the C-terminus, the underline indicates the linker, and the arrow indicates the SARS- Indicates the location where the CoV-2-B.1.1.351 Spike protein is inserted.

ATGGGCCTGCCCAACAACACCGCCAGCTGGTTTACCGCCCTGACCCAGCACGGC

AAGGAGGACCTGAAGTTCCCCAGAGGCCAGGGCGTGCCCATTAACACCAACAGC

AGCCCCGACGACCAGATCGGCTACTACAGAAGAGCCACCAGAAGAATCAGAGG

CGGCGACGGCAAGATGAAGGACCTGAGCCCCAGATGGTACTTCTACTATCTGGG

AACCGGCCCCGAAGCC

GGCCTGCCCTATGGCGCCAACAAAGATGGCATCATCTGGGTGGCCACCGAAGGC

GCCCTGAACACCCCCAAGGATCACATCGGAACCAGAAATCCCGCCAACAATGCC

GCCATCGTGCTGCAGCTGCCCCAGGGAACCACCCTGCCTAAAGGCTTTTATGCCG

AAGGAAGCAGAGGAGGAAGC (SEQ ID NO: 11)

GGCGGCGGAGGAAGCGGCGGAGGAGGCAGCGGCGGAGGCGGAAGC↓GGCGGAG

GAGGAAGCGGCGGAGGCGGAAGCGGCGGCGGAGGAAGCACCAAAAAGAGCGC

CGCCGAGGCCAGCAAAAAGCCCAGACAGAAAAGAACCGCCACCAAGGCCTACA

ATGTGACCCAGGCCTTTGGCAGAAGAGGACCCGAGCAGACCCAGGGCAATTTTG

GCGACCAGGAGCTGATCAGACAGGGCACCGACTACAAACACTGGCCCCAGATCG

CCCAGTTCGCCCCCAGCGCCAGCGCCTTCTTCGGCATGAGCAGAATCGGCATGGA

GGTGACCCCCAGCGGCACCTGGCTGACCTACACCGGCGCCATCAAGCTGGACGA

CAAGGACCCCAACTTCAAGGACCAGGTGATCCTGCTGAACAAGCACATCGACGC

CTACAAGACCTTCCCCTGA (SEQ ID NO: 12)

In the above sequence, SEQ ID NO: 11 is a gene sequence encoding a polypeptide bound to the N-terminus of the SARS-CoV-2-B. 1.1.351 Spike protein, and SEQ ID NO: 12 is a gene sequence encoding a polypeptide bound to the C-terminus, The underline indicates the gene sequence encoding the linker, and the arrow indicates the location where the SARS-CoV-2-B.1.1.351 Spike protein is inserted. [ChimAd vector-based HPV vaccine sequence]
SEQ ID NO: 13 - Human papillomavirus type 16, E6, E7 & Human papillomavirus type 18, E6, E7 modified amino acid sequence (linker underlined) 
MHQKRTAMFQDPQERPRKLPQLCTELQTTIHDIILECVYCKQQLLRREVYDFAFRDL

CIVYRDGNPYAVCDKCLKFYSKISEYRHYCYSLYGTTLEQQYNKPLCDLLIRCIKQR

HLDKKQRFHNIRGRWTGRCMSCCRSSRTRRETQLMHGDTPTLHEYMLDLQPETTQL

NDSSEEEDEIDGPAGQAEPDRAHYNIVTFCCKCDSTLRLCVQSTHVDIRTLEDLLMGT

LGIVCPICSQKPRGRKRRSMARFEDPTRRPYKLPDLCTELNTSLQDIEITCVYCKTVLE

LTEVFEFAFKDLFVVYRDSIPHAACHKCIDFYSRIRELRHYSDSVYGDTLEKLTNTGL

YNLLIRCLKLRHLNEKRRFHNIAGHYRGQCHSCCNRARQERLQRRRETQVMHGPKA

TLQDIVLHLEPQNEIPVQLSDSEEENDEIDGVNHQHLPARRAEPQRHTMLCMCCKCE

ARIELVVESSADDLRAFQQLFLNTLSFVCPWCASQQ
SEQ ID NO: 14 - Human papillomavirus type 16, E6, E7 & Human papillomavirus type 18, E6, E7 modified nucleic acid sequence (linker underlined)

ATGCACCAGAAGAGAACAGCCATGTTCCAGGACCCTCAGGAACGGCCACGGAAG
CTCCCACAACTTTGTACAGAGCTGCAAACAACCATTCATGACATCATCCTTGAAT
GCGTTTACTGCAAGCAGCAACTTCTGCGCCGGGAAGTCTACGATTTCGCTTTCAG
AGATCTGTGCATCGTGTATAGAGACGGGAACCCCTACGCCGTGTGTGACAAATG
CCTTAAATTCTACAGTAAGATTTCAGAGTACCGGCATTATTGCTACAGCCTTTAT
GGAACTACATTGGAGCAGCAGTACAACAAACCTTTGTGCGATCTTCTTATCCGGT
GCATTAAGCAACGGCACTTGGACAAAAAACAACGGTTCCATAACATCAGGGGCA
GGTGGACTGGACGCTGCATGAGTTGCTGTCGCTCAAGCCGCACTCGGAGAGAGA

CTCAACTGATGCATGGTGATACTCCAACTTTGCACGAATATATGTTGGACCTCCA
ACCCGAAACTACCCAACTGAATGACAGCTCTGAGGAAGAGGACGAAATCGACGG
CCCTGCAGGGCAAGCCGAACCTGACAGGGCCCATTATAACATTGTGACATTTTGC
TGCAAATGCGATAGTACTTTGAGACTCTGTGTGCAGTCAACTCATGTTGACATTA
GAACACTTGAAGACCTCCTCATGGGGACACTGGGGATTGTTTGCCCAATCTGTTC
ACAGAAACCCAGAGGCCGCAAAAGGCGGTCTATGGCTCGCTTTGAGGACCCTAC
AAGGAGGCCCTACAAGCTCCCCGATTTGTGTACAGAACTGAATACCAGTCTTCAG
GATATTGAAATCACTTGCGTCTACTGCAAAACTGTGCTCGAACTTACCGAAGTCT
TTGAATTTGCCTTCAAGGACCTTTTTGTTGTGTATCGCGATTCAATCCCACACGCA
GCCTGTCACAAATGTATTGACTTCTACTCTAGGATCAGAGAATTGAGGCACTATT
CAGACTCTGTGTATGGCGACACCTTGGAAAAATTGACCAACACTGGATTGTATAA
TCTGCTTATTCGGTGTCTTAAGCTCCGGCACTTGAACGAAAAACGCAGATTCCAC
AACATTGCCGGGCATTATCGCGGGCAATGCCATTCTTGTTGCAATCGCGCCCGCC
AGGAGCGGCTGCAAAGGCGCAGAGAGACTCAGGTGATGCATGGACCCAAGGCC
ACTCTCCAGGACATCGTCCTTCATCTTGAACCCCAGAACGAAATTCCCGTTCAAC
TTAGCGACTCAGAAGAAGAAAATGATGAAATCGACGGAGTGAACCATCAGCATC
TCCCAGCAAGAAGAGCCGAGCCACAAAGACACACAATGCTCTGTATGTGCTGCA
AGTGTGAAGCACGGATCGAACTCGTCGTCGAATCTAGTGCAGATGATCTCAGAG
CCTTTCAACAGCTCTTCTTGAATACACTGAGCTTTGTCTGTCCATGGTGCGCTAGT
CAACAA

## Claims

1. A chimeric adenovirus vector in which the knob domain of the end of the fiber protein of human adenovirus type 5 has been replaced with the knob gene of chimpanzee adenovirus serotype 6.

2. The chimeric adenovirus vector according to claim 1, wherein the hexon protein of human adenovirus type 5 is further replaced with hypervariable regions 1-7 of human adenovirus serotype 28.

3. The chimeric adenovirus vector according to claim 1, wherein the protein expressed by the knob gene of chimpanzee adenovirus serotype 6 comprises the amino acid sequence of SEQ ID NO: 1.

4. The chimeric adenovirus vector according to claim 1, wherein the knob gene sequence of chimpanzee adenovirus serotype 6 comprises the nucleic acid sequence of SEQ ID NO: 2.

5. The chimeric adenovirus vector according to claim 1, wherein the vector further comprises a fiber protein combined with the tail and shaft domains of human adenovirus type 5 and the knob domain of chimpanzee adenovirus serotype 6.

6. The chimeric adenovirus vector according to claim 2, wherein the chimeric protein in which the hexon protein of human adenovirus type 5 is replaced with the hypervariable region 1-7 of human adenovirus serotype 28 comprises the amino acid sequence of SEQ ID NO: 3.

7. The chimeric adenovirus vector according to claim 2, wherein the gene encoding a chimeric protein in which the hexon protein of human adenovirus type 5 is replaced with hypervariable regions 1-7 of human adenovirus serotype 28 comprises the nucleic acid sequence of SEQ ID NO: 4.

8. The chimeric adenovirus vector according to claim 1, wherein the vector is shown in Figure 6 or Figure 8.

9. The chimeric adenovirus vector of claim 1, wherein the vector comprises as an insert
a) a gene encoding the spike protein of the coronavirus, or
b) a gene encoding the E6 and E7 proteins of human papillomavirus type 16 or 18.

10. A host cell transformed with the chimeric adenovirus vector of any one of claims 1 to 9.

11. The host cell according to claim 10, wherein the cell is HEK293, PERC6 or 911 cell.

12. An isolated polypeptide encoded by the chimeric adenovirus vector of any one of claims 1 to 9.

13. A vaccine composition comprising the chimeric adenovirus vector of any one of claims 1 to 9 or a polypeptide encoded by the vector as an active ingredient.

14. A vaccine composition for preventing or treating SARS-CoV-2 infection comprising the chimeric adenovirus vector of any one of claims 1 to 9 or a polypeptide encoded by the vector as an active ingredient.

15. A vaccine composition for treating cervical cancer caused by human papillomavirus disease comprising the chimeric adenovirus vector of any one of claims 1 to 9 or a polypeptide encoded by the vector as an active ingredient.
